# EUROPEAN PATENT SPECIFICATION

(11) **EP 4 128 259 B1**
(45) Date of publication and mention of the grant of the patent: **30.07.2025**
(21) Application number: 22718795.2
(22) Date of filing: 11.04.2022
(51) Int. Cl.: G16H 20/40, G16H 40/63, G16H 40/67, G06F 3/01, G06V 20/20, A61B 1/12, A61B 1/00

(54) **ANTICIPATION OF INTERACTIVE UTILIZATION OF COMMON DATA OVERLAYS BY DIFFERENT USERS**
VORWEGNAHME EINER INTERAKTIVEN NUTZUNG VON GEMEINSAMEN DATENÜBERLAGERUNGEN DURCH VERSCHIEDENE BENUTZER
ANTICIPATION DE L'UTILISATION INTERACTIVE DE RECOUVREMENTS DE DONNÉES COMMUNES PAR DIFFÉRENTS UTILISATEURS

(30) Priority: 14.04.2021 US 202163174674 P; 30.11.2021 US 202163284326 P; 07.03.2022 US 202217688651
(43) Date of publication of application: 08.02.2023
(73) Proprietor: Cilag GmbH International, 6300 Zug (CH)
(72) Inventor: SHELTON, IV, Frederick E., Cincinnati, Ohio 45242 (US); ADAMS, Shane R., Cincinnati, Ohio 45242 (US); COWPERTHWAIT, Matthew D., Cincinnati, Ohio 45242 (US); VANOSDOLL, Madison K., Cincinnati, Ohio 45242 (US); KIMBALL, Cory G., Cincinnati, Ohio 45242 (US); RIVARD, Monica L.Z., Cincinnati, Ohio 45242 (US); ROSSONI, Leonardo N., Somerville, New Jersey 08876-0151 (US); KOJCEV, Risto, Santa Clara, California 95054 (US); BORK, Felix J., 20249 Hamburg (DE); BREHM, Tyler N., Cincinnati, Ohio 45242 (US); ECKERT, Chad E., Cincinnati, Ohio 45242 (US); BURKART, Ellen E., Cincinnati, Ohio 45242 (US)
(74) Representative: Carpmaels & Ransford LLP
(86) International application number: PCT/IB2022/053365
(87) International publication number: WO 2022/219493

(56) References cited:
- US-A1- 2013 038 707
- US-A1- 2016 066 184
- US-A1- 2019 206 565
- US-A1- 2020 015 895
- P. VÁVRA ET AL: "Recent Development of Augmented Reality in Surgery: A Review", JOURNAL OF HEALTHCARE ENGINEERING, vol. 2017, 4574172, 21 August 2017 (2017-08-21), Brentwood, pages 1 - 9, XP055520883, ISSN: 2040-2295, DOI: 10.1155/2017/4574172
- QIAN LONG ET AL: "A Review of Augmented Reality in Robotic-Assisted Surgery", IEEE TRANSACTIONS ON MEDICAL ROBOTICS AND BIONICS, IEEE, vol. 2, no. 1, 2 December 2019 (2019-12-02), pages 1 - 16, XP011773981, DOI: 10.1109/TMRB.2019.2957061

## Description

### BACKGROUND

This disclosure relates to apparatuses, systems, and methods for providing an augmented reality interactive experience during a surgical procedure. During a surgical procedure it would be desirable to provide an augmented reality interactive experience of a real-world environment where objects that reside in the real world are enhanced by overlaying computer-generated perceptual information, sometimes across multiple sensory modalities, including visual, auditory, haptic, somatosensory, and olfactory. In the context of this disclosure, images of a surgical field and surgical instruments and other objects appearing in the surgical field are enhanced by overlaying computer-generated visual, auditory, haptic, somatosensory, olfactory, or other sensory information onto the real world images of the surgical field and instruments or other objects appearing in the surgical field. The images may be streamed in real time or may be still images.

Real world surgical instruments include a variety of surgical devices including energy, staplers, or combined energy and staplers. Energy based medical devices include, without limitation, radio-frequency (RF) based monopolar and bipolar electrosurgical instruments, ultrasonic surgical instruments, combination RF electrosurgical and ultrasonic instruments, combination RF electrosurgical and mechanical staplers, among others. Surgical stapler devices are surgical instruments used to cut and staple tissue in a variety of surgical procedures, including bariatric, thoracic, colorectal, gynecologic, urologic and general surgery.

US 2016/0066184 describes an apparatus comprising a security engine to operate in a trusted execution environment to perform security operations and to authenticate a user of the apparatus, and to pair the device with a peer device depending on whether the devices are used by corresponding users.

US 2019/0206565 describes a method for adjusting the operation of a surgical instrument using machine learning in a surgical suite.

US 2013/0038707 describes a system and method for improving a surgeon's vision by overlaying augmented reality information onto a video image of the surgical site.

US 2020/0015895 describes a method comprising pre-registering an anatomical body part with a coordinate system used by an augmented reality device (such as augmented reality glasses) for outputting (e.g. displaying or projecting) augmentation information.

Recent Development of Augmented Reality in Surgery; A Review, P. Vávra et al., Journal of Healthcare Engineering 2017 Aug 21; doi: 10.1155/2017/4574172 evaluates whether augmented reality can presently improve the results of surgical procedures.

A Review of Augmented Reality in Robotic-Assisted Surgery, L. Qian et al., IEEE Transactions on Medical Robotics and Bionics 2020 Feb; doi: 10.1109/TMRB.2019.2957061 discusses the literature surrounding augmented reality in robotic-assisted surgery.

None of the above documents disclose a first interactive data overlay including an alert based on a detected error exceeding a risk level threshold.

### SUMMARY

The scope of the invention is defined by the appended claims 1-18. In various aspects, the present disclosure provides a method for displaying interactive overlays for multiple users of a surgical system. In some aspects, the method comprises receiving, by a surgical hub, a plurality of data streams related to a surgical procedure; communicably coupling a first augmented reality display device to a surgical hub; linking, by the surgical hub, the first augmented reality display device to a first user; and displaying, by the first augmented reality display device, a first interactive overlay customized for the first user based on at least one of the plurality of data streams.

In various aspects, the present disclosure provides a surgical system for displaying interactive overlays for multiple users. In some aspects, the system comprises a surgical hub configured to receive a plurality of data streams related to a surgical procedure; and a first augmented reality display device communicably coupled to the surgical hub. In one aspect, the first augment reality display device is linked to a first user. In another aspect, the first augmented reality display device displays a first interactive overlay customized for the first user based on at least one of the plurality of data streams.

### FIGURES

The various aspects described herein, both as to organization and methods of operation, together with further objects and advantages thereof, may best be understood by reference to the following description, taken in conjunction with the accompanying drawings as follows.
FIG. 1 is a block diagram of a computer-implemented interactive surgical system, according to one aspect of this disclosure.
FIG. 2 is a surgical system being used to perform a surgical procedure in an operating room, according to one aspect of this disclosure.
FIG. 3 is a surgical hub paired with a visualization system, a robotic system, and an intelligent instrument, according to one aspect of this disclosure.
FIG. 4 illustrates a surgical data network comprising a modular communication hub configured to connect modular devices located in one or more operating theaters of a healthcare facility, or any room in a healthcare facility specially equipped for surgical operations, to the cloud, according to one aspect of this disclosure.
FIG. 5 illustrates a computer-implemented interactive surgical system, according to one aspect of this disclosure.
FIG. 6 illustrates a surgical hub comprising a plurality of modules coupled to the modular control tower, according to one aspect of this disclosure.
FIG. 7 illustrates an augmented reality (AR) system comprising an intermediate signal combiner positioned in the communication path between an imaging module and a surgical hub display, according to one aspect of this disclosure.
FIG. 8 illustrates an augmented reality (AR) system comprising an intermediate signal combiner positioned in the communication path between an imaging module and a surgical hub display, according to one aspect of this disclosure.
FIG. 9 illustrates an augmented reality (AR) device worn by a surgeon to communicate data to the surgical hub, according to one aspect of this disclosure.
FIG. 10 illustrates a system for augmenting surgical instrument information using an augmented reality display, according to one aspect of this disclosure.
FIG. 11 illustrates a timeline of a situational awareness surgical procedure, according to one aspect of this disclosure.
FIG. 12 illustrates a surgical system configured to display interactive overlays for multiple users based on a plurality of data streams, according to one aspect of this disclosure.
FIG. 13 illustrates a method of displaying interactive overlays for multiple users of a surgical system, according to one aspect of this disclosure.
FIG. 14 illustrates a method for detecting a device-related error and determining actions to implement based on the detected error, according to one aspect of this disclosure.
FIGs. 15A, 15B, 15C, 15D, 15E, and 15F illustrate an exemplary implementation of the method of FIG. 14 during a thyroidectomy procedure, according to one aspect of this disclosure.
FIG. 16 illustrates a method for ensuring the secure wireless paring of smart devices to a surgical system, according to one aspect of this disclosure.
FIG. 17 illustrates a method for ensuring data authenticity and/or integrity after initial device pairing, according to one aspect of this disclosure.

Corresponding reference characters indicate corresponding parts throughout the several views. The exemplifications set out herein illustrate various disclosed embodiments, in one form, and such exemplifications are not to be construed as limiting the scope thereof in any manner.

### DESCRIPTION

Before explaining various aspects of surgical devices and generators in detail, it should be noted that the illustrative examples are not limited in application or use to the details of construction and arrangement of parts illustrated in the accompanying drawings and description. The illustrative examples may be implemented or incorporated in other aspects, variations and modifications, and may be practiced or carried out in various ways. Further, unless otherwise indicated, the terms and expressions employed herein have been chosen for the purpose of describing the illustrative examples for the convenience of the reader and are not for the purpose of limitation thereof. Also, it will be appreciated that one or more of the following-described aspects, expressions of aspects, and/or examples, can be combined with any one or more of the other following-described aspects, expressions of aspects and/or examples.

Various aspects are directed to onscreen displays for surgical systems for a variety of energy and surgical stapler based medical devices. Energy based medical devices include, without limitation, radio-frequency (RF) based monopolar and bipolar electrosurgical instruments, ultrasonic surgical instruments, combination RF electrosurgical and ultrasonic instruments, combination RF electrosurgical and mechanical staplers, among others. Surgical stapler devices include and combined surgical staplers with electrosurgical and / or ultrasonic devices. Aspects of the ultrasonic surgical devices can be configured for transecting and / or coagulating tissue during surgical procedures, for example. Aspects of the electrosurgical devices can be configured for transecting, coagulating, sealing, welding and / or desiccating tissue during surgical procedures, for example. Aspects of the surgical stapler devices can be configured for transecting and stapling tissue during surgical procedures and in some aspects, the surgical stapler devices may be configured to delivery RF energy to the tissue during surgical procedures. Electrosurgical devices are configured to deliver therapeutic and / or nontherapeutic RF energy to the tissue. Elements of surgical staplers, electrosurgical, and ultrasonic devices may be used in combination in a single surgical instrument.

In various aspects, the present disclosure provides onscreen displays of real time information to the OR team during a surgical procedure. In accordance with various aspects of the present disclosure, many new and unique onscreen displays are provided to display onscreen a variety of visual information feedback to the OR team. According to the present disclosure, visual information may comprise one or more than one of various visual media with or without sound. Generally, visual information comprises still photography, motion picture photography, video or audio recording, graphic arts, visual aids, models, display, visual presentation services, and the support processes. The visual information can be communicated on any number of display options such as the primary OR screen, the energy or surgical stapler device itself, a tablet, augmented reality glasses, among others, for example.

In various aspects, the present disclosure provides a large list of potential options to communicate visual information in real time to the OR team, without overwhelming the OR team with too much visual information. For example, in various aspects, the present disclosure provides onscreen displays of visual information to enable the surgeon, or other members of the OR team, to selectively activate onscreen displays such as icons surrounding the screen option to manage a wealth of visual information. One or a combination of factors can be used to determine the active display, these may include energy based (e.g., electrosurgical, ultrasonic) or mechanical based (e.g., staplers) surgical devices in use, the estimated risk associated with a given display, the experience level of the surgeon and the surgeons' choice among other things. In other aspect, the visual information may comprises rich data overlaid or superimposed into the surgical field of view to manage the visual information. In various aspects described hereinbelow, comprise superimposed imagery that requires video analysis and tracking to properly overlay the data. Visual information data communicated in this manner, as opposed to static icons, may provide additional useful visual information in a more concise and easy to understand way to the OR team.

In various aspects, the present disclosure provides techniques for selectively activating onscreen displays such as icons surrounding the screen to manage visual information during a surgical procedure. In other aspects, the present disclosure provides techniques for determining the active display using one or a combination of factors. In various aspects, the techniques according to the resent disclosure may comprise selecting the energy based or mechanical based surgical device in use as the active display, estimating risk associated with a given display, utilizing the experience level of the surgeon or OR team making the selection, among other things.

In other aspects, the techniques according to the present disclosure may comprise overlaying or superimposing rich data onto the surgical field of view to manage the visual information. A number of the display arrangements described by the present disclosure involve overlaying various visual representations of surgical data onto a livestream of a surgical field. As used herein the term overlay comprises a translucent overlay, a partial overlay, and / or a moving overlay. Graphical overlays may be in the form of a transparent graphic, semitransparent graphic, or opaque graphic, or a combination of transparent, semitransparent, and opaque elements or effects. Moreover, the overlay can be positioned on, or at least partially on, or near an object in the surgical field such as, for example, an end effector and/or a critical surgical structure. Certain display arrangements may comprise a change in one or more display elements of an overlay including a change in color, size, shape, display time, display location, display frequency, highlighting, or a combination thereof, based on changes in display priority values. The graphical overlays are rendered on top of the active display monitor to convey important information quickly and efficiently to the OR team.

In other aspects, the techniques according to the present disclosure may comprise superimposing imagery that requires analyzing video and tracking for properly overlaying the visual information data. In other aspects, the techniques according to the present disclosure may comprise communicating rich visual information, as opposed to simple static icons, to provide additional visual information to the OR team in a more concise and easy to understand manner. In other aspects, the visual overlays may be used in combination with audible and / or somatosensory overlays such as thermal, chemical, and mechanical devices, and combinations thereof.

The following description is directed generally to apparatuses, systems, and methods that provide an augmented reality (AR) interactive experience during a surgical procedure. In this context, images of a surgical field and surgical instruments and other objects appearing in the surgical field are enhanced by overlaying computer-generated visual, auditory, haptic, somatosensory, olfactory, or other sensory information onto the real world images of the surgical field, instruments, and/or other objects appearing in the surgical field. The images may be streamed in real time or may be still images. Augmented reality is a technology for rendering and displaying virtual or "augmented" virtual objects, data, or visual effects overlaid on a real environment. The real environment may include a surgical field. The virtual objects overlaid on the real environment may be represented as anchored or in a set position relative to one or more aspects of the real environment. In a non-limiting example, if a real world object exits the real environment field of view, a virtual object anchored to the real world object would also exit the augmented reality field of view.

A number of the display arrangements described by the present disclosure involve overlaying various visual representations of surgical data onto a livestream of a surgical field. As used herein the term overlaying comprises a translucent overlay, a partial overlay, and/or a moving overlay. Moreover, the overlay can be positioned on, or at least partially on, or near an object in the surgical field such as, for example, an end effector and/or a critical surgical structure. Certain display arrangements may comprise a change in one or more display elements of an overlay including a change in color, size, shape, display time, display location, display frequency, highlighting, or a combination thereof, based on changes in display priority values.

As described herein AR is an enhanced version of the real physical world that is achieved through the use of digital visual elements, sound, or other sensory stimuli delivered via technology. Virtual Reality (VR) is a computer-generated environment with scenes and objects that appear to be real, making the user feel they are immersed in their surroundings. This environment is perceived through a device known as a Virtual Reality headset or helmet. Mixed reality (MR) and AR are both considered immersive technologies, but they aren't the same. MR is an extension of Mixed reality that allows real and virtual elements to interact in an environment. While AR adds digital elements to a live view often by using a camera, an MR experience combines elements of both AR and VR, where real-world and digital objects interact.

In an AR environment, one or more computer-generated virtual objects may be displayed along with one or more real (i.e., so-called "real world") elements. For example, a real-time image or video of a surrounding environment may be shown on a computer screen display with one or more overlaying virtual objects. Such virtual objects may provide complementary information relating to the environment or generally enhance a user's perception and engagement with the environment. Conversely, the real-time image or video of the surrounding environment may additionally or alternatively enhance a user's engagement with the virtual objects shown on the display.

The apparatuses, systems, and methods in the context of this disclosure enhance images received from one or more imaging devices during a surgical procedure. The imaging devices may include a variety of scopes used during non-invasive and minimally invasive surgical procedures, an AR device, and/or a camera to provide images during open surgical procedures. The images may be streamed in real time or may be still images. The apparatuses, systems, and methods provide an augmented reality interactive experience by enhancing images of the real world surgical environment by overlaying virtual objects or representations of data and/or real objects onto the real surgical environment. The augmented reality experience may be viewed on a display and/or an AR device that allows a user to view the overlaid virtual objects onto the real world surgical environment. The display may be located in the operating room or remote from the operating room. AR devices are worn on the head of the surgeon or other operating room personnel and typically include two stereo-display lenses or screens, including one for each eye of the user. Natural light is permitted to pass through the two transparent or semi-transparent display lenses such that aspects of the real environment are visible while also projecting light to make virtual objects visible to the user of the AR device.

Two or more displays and AR devices may be used in a coordinated manner, for example with a first display or AR device controlling one or more additional displays or AR devices in a system with defined roles. For example, when activating display or an AR device, a user may select a role (e.g., surgeon, surgical assistant, nurse, etc., during a surgical procedure) and the display or AR device may display information relevant to that role. For example, a surgical assistant may have a virtual representation of an instrument displayed that the surgeon needs to perform for a next step of a surgical procedure. A surgeon's focus on the current step may see different information displayed than the surgical assistant.

Although there are many known onscreen displays and alerts, this disclosure provides many new and unique augmented reality interactive experiences during a surgical procedure. Such augmented reality interactive experiences include visual, auditory, haptic, somatosensory, olfactory, or other sensory feedback information to the surgical team inside or outside the operating room. The virtual feedback information overlaid onto the real world surgical environment may be provided to an operating room (OR) team, including personnel inside the OR including, without limitation, the operating surgeon, assistants to the surgeon, a scrub person, an anesthesiologist and a circulating nurse, among others, for example. The virtual feedback information can be communicated on any number of display options such as a primary OR screen display, an AR device, the energy or surgical stapler instrument, a tablet, augmented reality glasses, device etc.

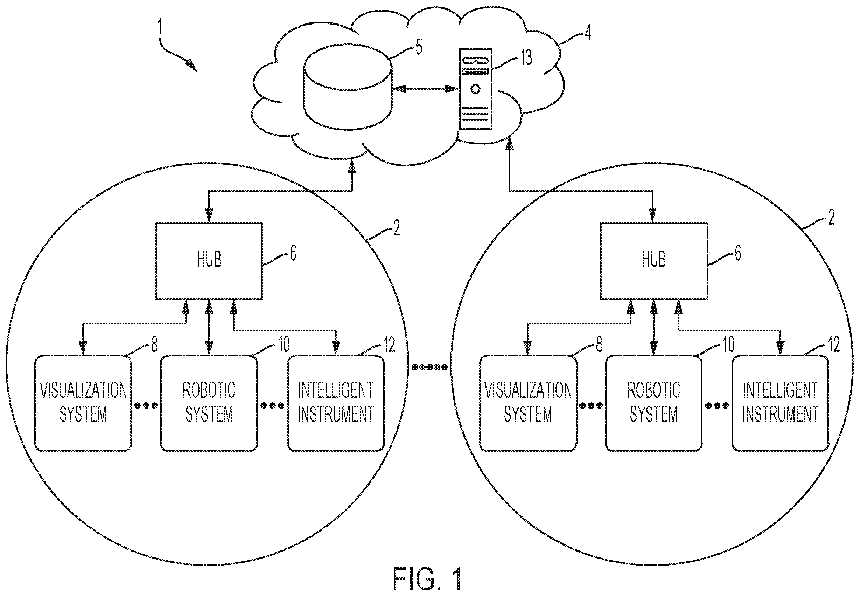

FIG. 1 depicts a computer-implemented interactive surgical system 1 that includes one or more surgical systems 2 and a cloud-based system 4. The cloud-based system 4 may include a remote server 13 coupled to a storage device 5. Each surgical system 2 includes at least one surgical hub 6 in communication with the cloud 4. For example, the surgical system 2 may include a visualization system 8, a robotic system 10, and handheld intelligent surgical instruments 12, each configured to communicate with one another and/or the hub 6. In some aspects, a surgical system 2 may include an M number of hubs 6, an N number of visualization systems 8, an O number of robotic systems 10, and a P number of handheld intelligent surgical instruments 12, where M, N, O, and P are integers greater than or equal to one. The computer-implemented interactive surgical system 1 may be configured to provide an augmented reality interactive experience during a surgical procedure as described herein.

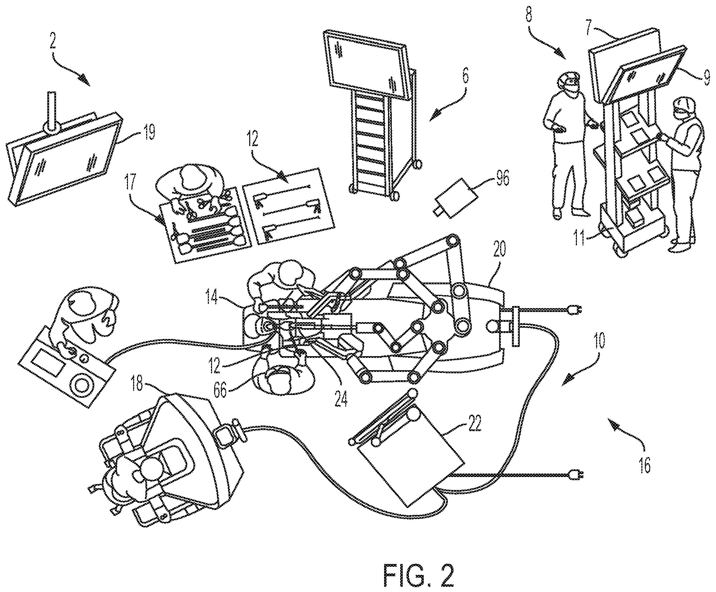

FIG. 2 depicts an example of a surgical system 2 to perform a surgical procedure on a patient lying down on an operating table 14 in a surgical operating room 16. A robotic system 10 is used in the surgical procedure as a part of the surgical system 2. The robotic system 10 includes a surgeon's console 18, a patient side cart 20 (surgical robot), and a surgical robotic hub 22. The patient side cart 20 can manipulate at least one removably coupled surgical tool 17 through a minimally invasive incision in the body of the patient while the surgeon views the surgical site through the surgeon's console 18 or an augmented reality (AR) device 66 worn by the surgeon. An image (e.g., still or live streamed in real time) of the surgical site during a minimally invasive procedure can be obtained by a medical imaging device 24. The patient side cart 20 can manipulate the imaging device 24 to orient the imaging device 24. An image of an open surgical procedure can be obtained by a medical imaging device 96. The robotic hub 22 processes the images of the surgical site for subsequent display on the surgeon's console 18 or the AR device 66 worn by the surgeon, or other person in the surgical operating room 16.

The optical components of the imaging device 24, 96 or AR device 66 may include one or more illumination sources and/or one or more lenses. The one or more illumination sources may be directed to illuminate portions of the surgical field. One or more image sensors may receive light reflected or refracted from tissue and instruments in the surgical field.

In various aspects, the imaging device 24 is configured for use in a minimally invasive surgical procedure. Examples of imaging devices suitable for use with this disclosure include, but not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope. In various aspects, the imaging device 96 is configured for use in an open (invasive) surgical procedure.

In various aspects, the visualization system 8 includes one or more imaging sensors, one or more image-processing units, one or more storage arrays, and one or more displays that are strategically arranged with respect to the sterile field. In one aspect, the visualization system 8 includes an interface for HL7, PACS, and EMR. In one aspect, the imaging device 24 may employ multi-spectrum monitoring to discriminate topography and underlying structures. A multispectral image captures image data within specific wavelength ranges in the electromagnetic spectrum. Wavelengths are separated by filters or instruments sensitive to particular wavelengths, including light from frequencies beyond the visible light range, e.g., IR and ultraviolet. Spectral imaging can extract information not visible to the human eye. Multi-spectrum monitoring can relocate a surgical field after a surgical task is completed to perform tests on the treated tissue.

FIG. 2 depicts a primary display 19 positioned in the sterile field to be visible to an operator at the operating table 14. A visualization tower 11 is positioned outside the sterile field and includes a first non-sterile display 7 and a second non-sterile display 9, which face away from each other. The visualization system 8, guided by the hub 6, is configured to utilize the displays 7, 9, 19 to coordinate information flow to operators inside and outside the sterile field. For example, the hub 6 may cause the visualization system 8 to display AR images of the surgical site, as recorded by an imaging device 24, 96 on a non-sterile display 7, 9, or through the AR device 66, while maintaining a live feed of the surgical site on the primary display 19 or the AR device 66. The non-sterile display 7, 9 can permit a non-sterile operator to perform a diagnostic step relevant to the surgical procedure, for example.

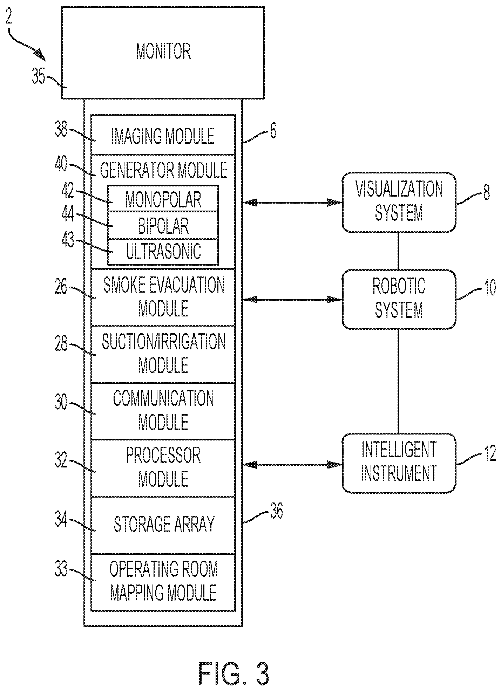

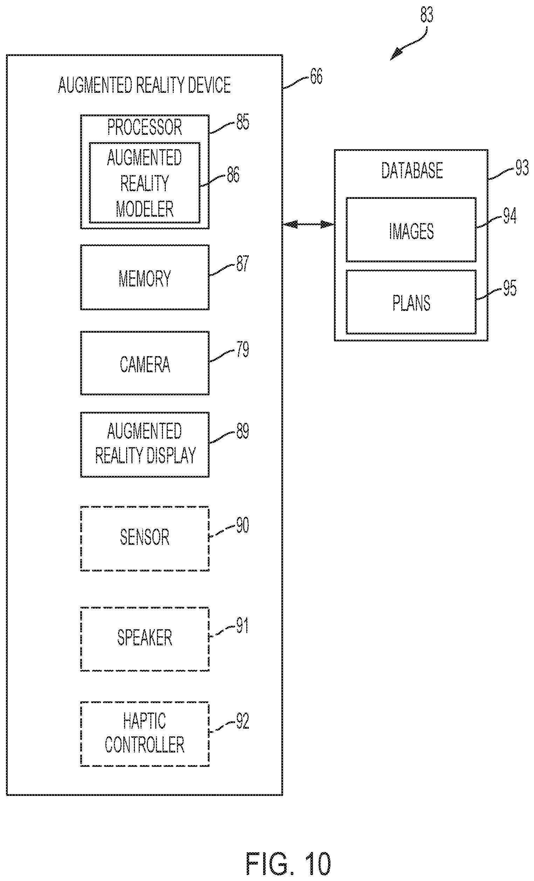

FIG. 3 depicts a hub 6 in communication with a visualization system 8, a robotic system 10, and a handheld intelligent surgical instrument 12. The hub 6 includes a hub display 35, an imaging module 38, a generator module 40, a communication module 30, a processor module 32, a storage array 34, and an operating room mapping module 33. The hub 6 further includes a smoke evacuation module 26 and/or a suction/irrigation module 28. In various aspects, the imaging module 38 comprises an AR device 66 and the processor module 32 comprises an integrated video processor and an augmented reality modeler (e.g., as shown in FIG. 10). A modular light source may be adapted for use with various imaging devices. In various examples, multiple imaging devices may be placed at different positions in the surgical field to provide multiple views (e.g., non-invasive, minimally invasive, invasive or open surgical procedures). The imaging module 38 can be configured to switch between the imaging devices to provide an optimal view. In various aspects, the imaging module 38 can be configured to integrate the images from the different imaging devices and provide an augmented reality interactive experience during a surgical procedure as described herein.

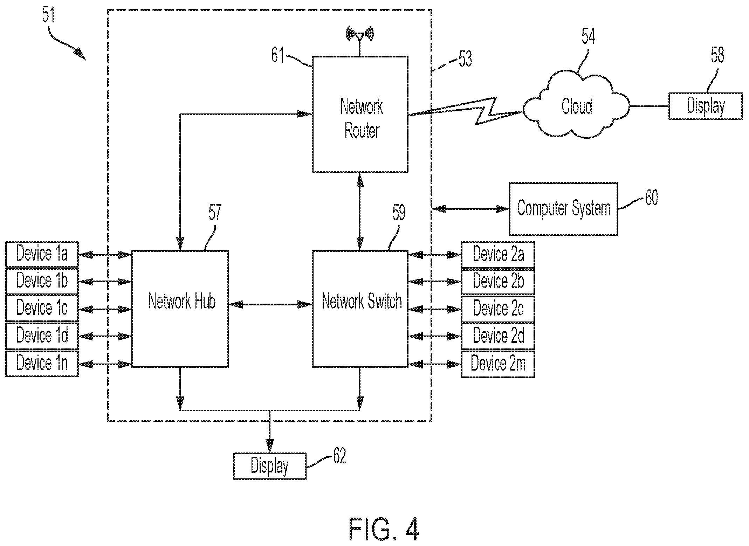

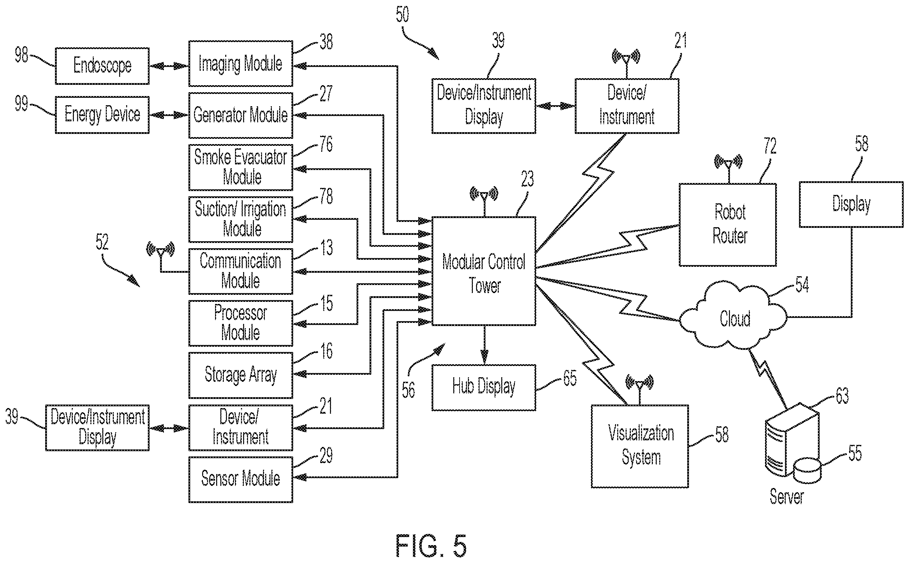

FIG. 4 shows a surgical data network 51 comprising a modular communication hub 53 configured to connect modular devices located in one or more operating theaters/rooms of a healthcare facility to a cloud-based system. The cloud 54 may include a remote server 63 (FIG. 5) coupled to a storage device 55. The modular communication hub 53 comprises a network hub 57 and/or a network switch 59 in communication with a network router 61. The modular communication hub 53 is coupled to a local computer system 60 to process data. Modular devices 1a-1n in the operating theater may be coupled to the modular communication hub 53. The network hub 57 and/or the network switch 59 may be coupled to a network router 61 to connect the devices 1a-1n to the cloud 54 or the local computer system 60. Data associated with the devices 1a-1n may be transferred to cloud-based computers via the router for remote data processing and manipulation. The operating theater devices 1a-1n may be connected to the modular communication hub 53 over a wired channel or a wireless channel. The surgical data network 51 environment may be employed to provide an augmented reality interactive experience during a surgical procedure as described herein and in particular providing augmented images if the surgical field to one or more than one remote display 58.

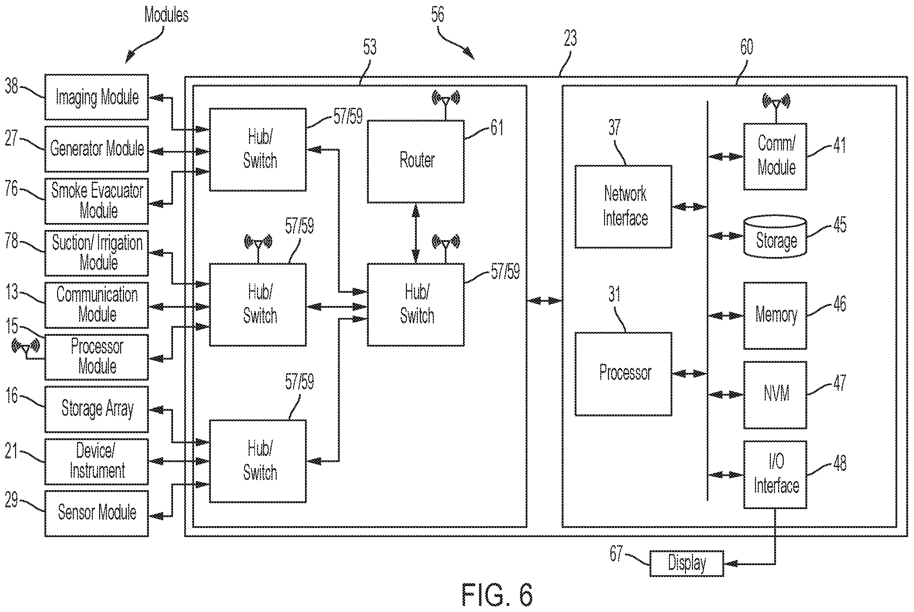

FIG. 5 illustrates a computer-implemented interactive surgical system 50. The computer-implemented interactive surgical system 50 is similar in many respects to the computer-implemented interactive surgical system 1. The computer-implemented interactive surgical system 50 includes one or more surgical systems 52, which are similar in many respects to the surgical systems 2. Each surgical system 52 includes at least one surgical hub 56 in communication with a cloud 54 that may include a remote server 63. In one aspect, the computer-implemented interactive surgical system 50 comprises a modular control tower 23 connected to multiple operating theater devices such as, for example, intelligent surgical instruments, robots, and other computerized devices located in the operating theater. As shown in FIG. 6, the modular control tower 23 comprises a modular communication hub 53 coupled to a computer system 60.

Back to FIG. 5, the modular control tower 23 is coupled to an imaging module 38 that is coupled to an endoscope 98, a generator module 27 that is coupled to an energy device 99, a smoke evacuator module 76, a suction/irrigation module 78, a communication module 13, a processor module 15, a storage array 16, a smart device/instrument 21 optionally coupled to a display 39, and a sensor module 29. The operating theater devices are coupled to cloud computing resources such as server 63, data storage 55, and displays 58 via the modular control tower 23. A robot hub 72 also may be connected to the modular control tower 23 and to the servers 63, data storage 55, and displays 58. The devices/instruments 21, visualization systems 58, among others, may be coupled to the modular control tower 23 via wired or wireless communication standards or protocols, as described herein. The modular control tower 23 may be coupled to a hub display 65 (e.g., monitor, screen) to display augmented images received comprising overlaid virtual objects on the real surgical field received from the imaging module 38, device/instrument display 39, and/or other visualization systems 58. The hub display 65 also may display data received from devices connected to the modular control tower 23 in conjunction with images and overlaid images.

FIG. 6 illustrates a surgical hub 56 comprising a plurality of modules coupled to the modular control tower 23. The modular control tower 23 comprises a modular communication hub 53, e.g., a network connectivity device, and a computer system 60 to provide local processing, visualization, and imaging of augmented surgical information, for example. The modular communication hub 53 may be connected in a tiered configuration to expand the number of modules (e.g., devices) that may be connected to the modular communication hub 53 and transfer data associated with the modules to the computer system 60, cloud computing resources, or both. Each of the network hubs/switches 57, 59 in the modular communication hub 53 may include three downstream ports and one upstream port. The upstream network hub/switch 57, 59 is connected to a processor 31 to provide a communication connection to the cloud computing resources and a local display 67. Communication to the cloud 54 may be made either through a wired or a wireless communication channel.

The computer system 60 comprises a processor 31 and a network interface 37. The processor 31 is coupled to a communication module 41, storage 45, memory 46, non-volatile memory 47, and input/output interface 48 via a system bus. The system bus can be any of several types of bus structure(s) including the memory bus or memory controller, a peripheral bus or external bus, and/or a local bus using any variety of available bus architectures.

The processor 31 comprises an augmented reality modeler (e.g., as shown in FIG. 10) and may be implemented as a single-core or multicore processor such as those known under the trade name ARM Cortex by Texas Instruments. In one aspect, the processor may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle serial random access memory (SRAM), an internal read-only memory (ROM) loaded with StellarisWare^{®} software, a 2 KB electrically erasable programmable read-only memory (EEPROM), and/or one or more pulse width modulation (PWM) modules, one or more quadrature encoder inputs (QEI) analogs, one or more 12-bit analog-to-digital converters (ADCs) with 12 analog input channels, details of which are available for the product datasheet.

The system memory includes volatile memory and non-volatile memory. The basic input/output system (BIOS), containing the basic routines to transfer information between elements within the computer system, such as during start-up, is stored in non-volatile memory. For example, the non-volatile memory can include ROM, programmable ROM (PROM), electrically programmable ROM (EPROM), EEPROM, or flash memory. Volatile memory includes random-access memory (RAM), which acts as external cache memory. Moreover, RAM is available in many forms such as SRAM, dynamic RAM (DRAM), synchronous DRAM (SDRAM), double data rate SDRAM (DDR SDRAM), enhanced SDRAM (ESDRAM), Synchlink DRAM (SLDRAM), and direct Rambus RAM (DRRAM).

The computer system 60 also includes removable/non-removable, volatile/non-volatile computer storage media, such as for example disk storage. The disk storage includes, but is not limited to, devices like a magnetic disk drive, floppy disk drive, tape drive, Jaz drive, Zip drive, LS-60 drive, flash memory card, or memory stick. In addition, the disk storage can include storage media separately or in combination with other storage media including, but not limited to, an optical disc drive such as a compact disc ROM device (CD-ROM), compact disc recordable drive (CD-R Drive), compact disc rewritable drive (CD-RW Drive), or a digital versatile disc ROM drive (DVD-ROM). To facilitate the connection of the disk storage devices to the system bus, a removable or non-removable interface may be employed.

In various aspects, the computer system 60 of FIG. 6, the imaging module 38 and/or visualization system 58, and/or the processor module 15 of FIGS. 4-6, may comprise an image processor, image-processing engine, graphics processing unit (GPU), media processor, or any specialized digital signal processor (DSP) used for the processing of digital images. The image processor may employ parallel computing with single instruction, multiple data (SIMD) or multiple instruction, multiple data (MIMD) technologies to increase speed and efficiency. The digital image-processing engine can perform a range of tasks. The image processor may be a system on a chip with multicore processor architecture.

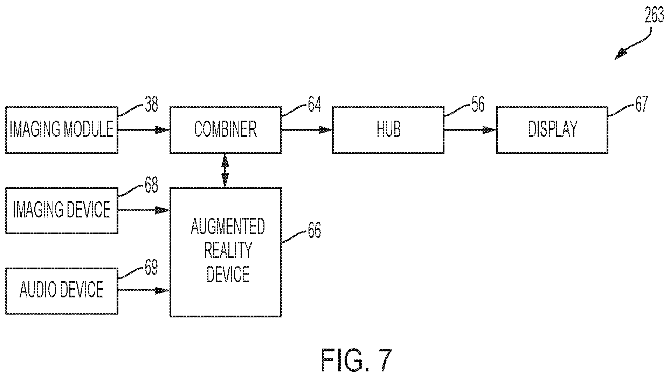

FIG. 7 illustrates an augmented reality system 263 comprising an intermediate signal combiner 64 positioned in the communication path between an imaging module 38 and a surgical hub display 67. The signal combiner 64 combines audio and/or image data received from an imaging module 38 and/or an AR device 66. The surgical hub 56 receives the combined data from the combiner 64 and overlays the data provided to the display 67, where the overlaid data is displayed. The imaging device 68 may be a digital video camera and the audio device 69 may be a microphone. The signal combiner 64 may comprise a wireless heads-up display adapter to couple to the AR device 66 placed into the communication path of the display 67 to a console allowing the surgical hub 56 to overlay data on the display 67.

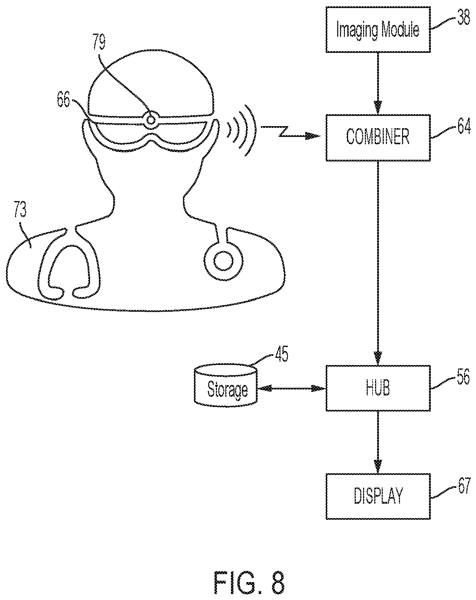

FIG. 8 illustrates an augmented reality (AR) system comprising an intermediate signal combiner positioned in the communication path between an imaging module and a surgical hub display. FIG. 8 illustrates an AR device 66 worn by a surgeon 73 to communicate data to the surgical hub 56. Peripheral information of the AR device 66 does not include active video. Rather, the peripheral information includes only device settings, or signals that do not have same demands of refresh rates. Interaction may augment the surgeon's 73 information based on linkage with preoperative computerized tomography (CT) or other data linked in the surgical hub 56. The AR device 66 can identify structure - ask whether instrument is touching a nerve, vessel, or adhesion, for example. The AR device 66 may include pre-operative scan data, an optical view, tissue interrogation properties acquired throughout procedure, and/or processing in the surgical hub 56 used to provide an answer. The surgeon 73 can dictate notes to the AR device 66 to be saved with patient data in the hub storage 45 for later use in report or in follow up.

The AR device 66 worn by the surgeon 73 links to the surgical hub 56 with audio and visual information to avoid the need for overlays, and allows customization of displayed information around periphery of view. The AR device 66 provides signals from devices (e.g., instruments), answers queries about device settings, or positional information linked with video to identify quadrant or position. The AR device 66 has audio control and audio feedback from the AR device 66. The AR device 66 is able to interact with other systems in the operating theater and have feedback and interaction available wherever the surgeon 73 is viewing. For example, the AR device 66 may receive voice or gesture initiated commands and queries from a surgeon, and the AR device 66 may provide feedback in the form of one or more modalities including audio, visual, or haptic touch.

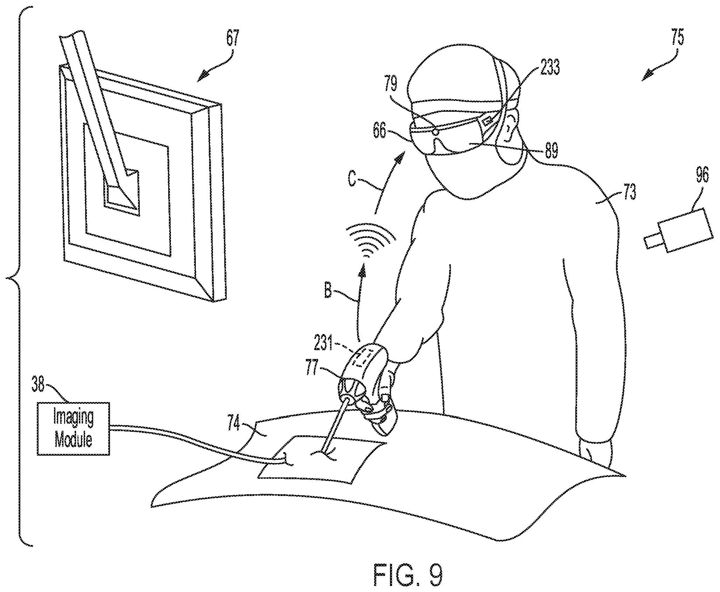

FIG. 9 illustrates a surgeon 73 wearing an AR device 66, a patient 74, and may include a camera 96 in an operating room 75. The AR device 66 worn by the surgeon 73 may be used to present to the surgeon 73 a virtual object overlaid on a real time image of the surgical field through augmented reality display 89 or through the hub connected display 67. The real time image may include a portion of a surgical instrument 77. The virtual object may not be visible to others within the operating room 75 (e.g., surgical assistant or nurse), though they also may wear AR devices 66. Even if another person is viewing the operating room 75 with an AR device 66, the person may not be able to see the virtual object or may be able to see the virtual object in a shared augmented reality with the surgeon 73, or may be able to see a modified version of the virtual object (e.g., according to customizations unique to the surgeon 73) or may see different virtual objects.

A virtual object and/or data may be configured to appear on a portion of a surgical instrument 77 or in a surgical field of view captured by an imaging module 38, an imaging device 68 during minimally invasive surgical procedures, and/or the camera 96 during open surgical procedures. In the illustrated example, the imaging module 38 is a laparoscopic camera that provides a live feed of a surgical area during a minimally invasive surgical procedure. An AR system may present virtual objects that are fixed to a real object without regard to a perspective of a viewer or viewers of the AR system (e.g., the surgeon 73). For example, a virtual object may be visible to a viewer of the AR system inside the operating room 75 and not visible to a viewer of the AR system outside the operating room 75. The virtual object may be displayed to the viewer outside the operating room 75 when the viewer enters the operating room 75. The augmented image may be displayed on the surgical hub display 67 or the augmented reality display 89.

The AR device 66 may include one or more screens or lens, such as a single screen or two screens (e.g., one per eye of a user). The screens may allow light to pass through the screens such that aspects of the real environment are visible while displaying the virtual object. The virtual object may be made visible to the surgeon 73 by projecting light. A virtual object may appear to have a degree of transparency or may be opaque (i.e., blocking aspects of the real environment).

An AR system may be viewable to one or more viewers, and may include differences among views available for the one or more viewers while retaining some aspects as universal among the views. For example, a heads-up display may change between two views while virtual objects and/or data may be fixed to a real object or area in both views. Aspects such as a color of an object, lighting, or other changes may be made among the views without changing a fixed position of at least one virtual object.

A user may see a virtual object and/or data presented in an AR system as opaque or as including some level of transparency. In an example, the user may interact with the virtual object, such as by moving the virtual object from a first position to a second position. For example, the user may move an object with his or her hand. This may be done in the AR system virtually by determining that the hand has moved into a position coincident or adjacent to the object (e.g., using one or more cameras, which may be mounted on the AR device 66, such as AR device camera 79 or separate 96, and which may be static or may be controlled to move), and causing the object to move in response. Virtual aspects may include virtual representations of real world objects or may include visual effects, such as lighting effects, etc. The AR system may include rules to govern the behavior of virtual objects, such as subjecting a virtual object to gravity or friction, or may include other predefined rules that defy real world physical constraints (e.g., floating objects, perpetual motion, etc.). The AR device 66 may include a camera 79 on the AR device 66 (not to be confused with the camera 96, separate from the AR device 66). The AR device camera 79 or the camera 96 may include an infrared camera, an infrared filter, a visible light filter, a plurality of cameras, a depth camera, etc. The AR device 66 may project virtual items over a representation of a real environment, which may be viewed by a user.

The AR device 66 may be used in the operating room 75 during a surgical procedure, for example performed by the surgeon 73 on the patient 74. The AR device 66 may project or display virtual objects, such as a virtual object during the surgical procedure to augment the surgeon's vision. The surgeon 73 may view a virtual object using the AR device 66, a remote controller for the AR device 66, or may interact with a virtual object, for example, using a hand to "interact" with a virtual object or a gesture recognized by the camera 79 of the AR device 66. A virtual object may augment a surgical tool such as the surgical instrument 77. For example, the virtual object may appear (to the surgeon 73 viewing the virtual object through the AR device 66) to be coupled with or remain a fixed distance from the surgical instrument 77. In another example, the virtual object may be used to guide the surgical instrument 77, and may appear to be fixed to the patient 74. In certain examples, a virtual object may react to movements of other virtual or real-world objects in the surgical field. For example, the virtual object may be altered when a surgeon is manipulating a surgical instrument in proximity to the virtual object.

The augmented reality display system imaging device 38 capture a real image of a surgical area during a surgical procedure. An augmented reality display 89, 67 presents an overlay of an operational aspect of the surgical instrument 77 onto the real image of the surgical area. The surgical instrument 77 includes communications circuitry 231 to communicate operational aspects and functional data from the surgical instrument 77 to the AR device 66 via communication communications circuitry 233 on the AR device 66. Although the surgical instrument 77 and the AR device 66 are shown in RF wireless communication between circuits 231, 233 as indicated by arrows B, C, other communication techniques may employed (e.g., wired, ultrasonic, infrared, etc.). The overlay is related to the operational aspect of the surgical instrument 77 being actively visualized. The overlay combines aspects of tissue interaction in the surgical area with functional data from the surgical instrument 77. A processor portion of the AR device 66 is configured to receive the operational aspects and functional data from the surgical instrument 77, determine the overlay related to the operation of the surgical instrument 77, and combine the aspect of the tissue in the surgical area with the functional data from the surgical instrument 77. The augmented images indicate alerts relative to device performance considerations, alerts of incompatible usage, alerts on incomplete capture. Incompatible usage includes tissue out range conditions and tissue incorrectly balanced within the jaws of the end effector. Additional augmented images provide an indication of collateral events including indication of tissue tension and indication of foreign object detection. Other augmented images indicate device status overlays and instrument indication.

FIG. 10 illustrates a system 83 for augmenting images of a surgical field with information using an AR display 89, in accordance with at least one aspect of this disclosure. The system 83 may be used to perform the techniques described hereinbelow, for example, by using the processor 85. The system 83 includes one aspect of an AR device 66 that may be in communication with a database 93. The AR device 66 includes a processor 85, memory 87, an AR display 89, and a camera 79. The AR device 66 may include a sensor 90, a speaker 91, and/or a haptic controller 92. The database 93 may include image storage 94 or preoperative plan storage 95.

The processor 85 of the AR device 66 includes an augmented reality modeler 86. The augmented reality modeler 86 may be used by the processor 85 to create the augmented reality environment. For example, the augmented reality modeler 86 may receive images of the instrument in a surgical field, such as from the camera 79 or sensor 90, and create the augmented reality environment to fit within a display image of the surgical field of view. In another example, physical objects and/or date may be overlaid on the surgical field of view and/or the surgical instruments images and the augmented reality modeler 86 may use physical objects and data to present the augmented reality display of virtual object s and/or data in the augmented reality environment. For example, the augmented reality modeler 86 may use or detect an instrument at a surgical site of the patient and present a virtual object and/or data on the surgical instrument and/or an image of the surgical site in the surgical field of view captured by the camera 79. The AR display 89 may display the AR environment overlaid on a real environment. The display 89 may show a virtual object and/or data, using the AR device 66, such as in a fixed position in the AR environment.

The AR device 66 may include a sensor 90, such as an infrared sensor. The camera 79 or the sensor 90 may be used to detect movement, such as a gesture by a surgeon or other user, that may be interpreted by the processor 85 as attempted or intended interaction by the user with the virtual target. The processor 85 may identify an object in a real environment, such as through processing information received using the camera 79. In other aspects, the sensor 90 may be a tactile, audible, chemical, or thermal sensor to generate corresponding signals that may combined with various data feeds to create the augmented environment. The sensor 90 may include binaural audio sensors (spatial sound), inertial measurement (accelerometer, gyroscope, magnetometer) sensors, environmental sensors, depth camera sensors, hand and eye tracking sensors, and voice command recognition functions.

The AR display 89, for example during a surgical procedure, may present, such as within a surgical field while permitting the surgical field to be viewed through the AR display 89, a virtual feature corresponding to a physical feature hidden by an anatomical aspect of a patient. The virtual feature may have a virtual position or orientation corresponding to a first physical position or orientation of the physical feature. In an example, the virtual position or orientation of the virtual feature may include an offset from the first physical position or orientation of the physical feature. The offset may include a predetermined distance from the augmented reality display, a relative distance from the augmented reality display to the anatomical aspect, or the like.

In one example, the AR device 66 may be an individual AR device. In one aspect, the AR device 66 may be a HoloLens 2 AR device manufactured by Microsoft of Redmond, Wash. This AR device 66 includes a visor with lenses and binaural audio features (spatial sound), inertial measurement (accelerometer, gyroscope, magnetometer), environmental sensors, depth camera, and video camera, hand and eye tracking, and voice command recognition functions. It provides an improved field of view with high resolution by using mirrors to direct waveguides in front of wearer's eyes. Images can be enlarged by changing angles of mirrors. It also provides eye tracking to recognize users and adjust lens widths for specific users.

In another example, the AR device 66 may be a Snapchat Spectacles 3 AR device. This AR device provides the ability to capture paired images and recreate 3D depth mapping, add in virtual effects, and replay 3D videos. The AR device includes two HD cameras to capture 3D photos and videos at 60 fps - while four built-in microphones record immersive, high-fidelity audio. Images from both cameras combine to build out a geometric map of the real world around the user to provide a new sense of depth perception. Photos and videos may be wirelessly synchronized to external display devices.

In yet another example, the AR device 66 may be a Glass 2 AR device by Google. This AR device provides inertial measurement (accelerometer, gyroscope, magnetometer) information overlaid on lens (out of view) to supplement information.

In another example, the AR device 66 may be an Echo Frames AR device by Amazon. This AR device does not have cameras/displays. A microphone and speaker are linked to Alexa. This AR device provides less functionality than a heads-up display.

In yet another example, the AR device 66 may be a Focals AR device by North (Google). This AR device provides notification pusher/smartwatch analog; inertial measurement, screen overlay of information (weather, calendar, messages), voice control (Alexa) integration. This AR device provides basic heads-up display functionality.

In another example, the AR device 66 may be an Nreal AR device. This AR device includes spatial sound, two environmental cameras, a photo camera, IMU (accelerometer, gyroscope), ambient light sensor, proximity sensor functionality. A nebula projects application information on lenses.

In various other examples, the AR device 66 may be any one of the following commercially available AR devices: Magic Leap 1, Epson Moverio, Vuzix Blade AR, ZenFone AR, Microsoft AR glasses prototype, EyeTap to create collinear light to that of the environment directly into the retina. A beam splitter makes the same light seen by the eye available to the computer to process and overlay information, for example. AR visualization systems include HUD, contact lenses , glasses, virtual reality (VR) headsets, virtual retinal display, on in operating room displays, and/or smart contact lenses (bionic lenses).

Multi-user interfaces for the AR device 66 include virtual retinal displays such as raster displays drawn directly on retinas instead of on a screen in front of the eye, smart televisions, smart phones, and / or spatial displays such as Sony spatial display systems.

Other AR technology may include, for example, AR capture devices and software applications, AR creation devices and software applications, and AR cloud devices and software applications. AR capture devices and software applications include, for example, Apple Polycam app, Ubiquity 6 (Mirrorworld using Display.land app) - users can scan and get 3d image of real world (to create 3D model). AR creation devices and software applications include, for example, Adobe Aero, Vuforia, ARToolKit, Google ARCore, Apple ARKit, MAXST, Aurasma, Zappar, Blippar. AR cloud devices and software applications include, for example, Facebook, Google (world geometry, objection recognition, predictive data), Amazon AR Cloud (commerce), Microsoft Azure, Samsung Project Whare, Niantic, Magic Leap.

Situational awareness is the ability of some aspects of a surgical system to determine or infer information related to a surgical procedure from data received from databases and/or instruments. The information can include the type of procedure being undertaken, the type of tissue being operated on, or the body cavity that is the subject of the procedure. With the contextual information related to the surgical procedure, the surgical system can, for example, improve the manner in which it controls the modular devices (e.g., a robotic arm and/or robotic surgical tool) that are connected to it and provide contextualized information or suggestions to the surgeon during the course of the surgical procedure.

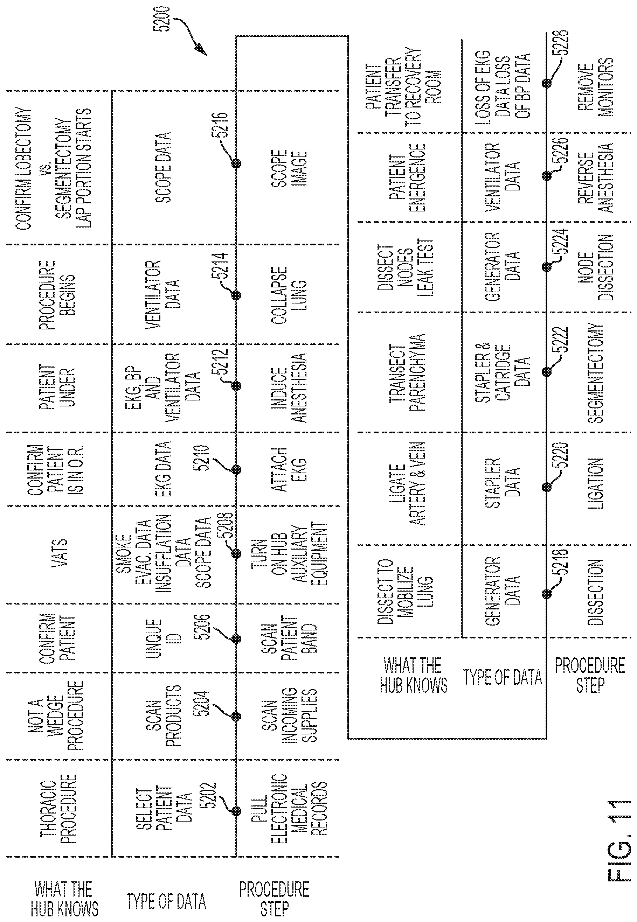

FIG. 11 illustrates a timeline of a situational awareness surgical procedure. FIG. 11 illustrates a timeline 5200 of an illustrative surgical procedure and the contextual information that a surgical hub 5104 can derive from the data received from the data sources 5126 at each step in the surgical procedure. The timeline 5200 depicts the typical steps that would be taken by the nurses, surgeons, and other medical personnel during the course of a lung segmentectomy procedure, beginning with setting up the operating theater and ending with transferring the patient to a post-operative recovery room. The situationally aware surgical hub 5104 receives data from the data sources 5126 throughout the course of the surgical procedure, including data generated each time medical personnel utilize a modular device 5102 that is paired with the surgical hub 5104. The surgical hub 5104 can receive this data from the paired modular devices 5102 and other data sources 5126 and continually derive inferences (i.e., contextual information) about the ongoing procedure as new data is received, such as which step of the procedure is being performed at any given time. The situational awareness system of the surgical hub 5104 is able to, for example, record data pertaining to the procedure for generating reports, verify the steps being taken by the medical personnel, provide data or prompts (e.g., via a display screen) that may be pertinent for the particular procedural step, adjust modular devices 5102 based on the context (e.g., activate monitors, adjust the FOV of the medical imaging device, or change the energy level of an ultrasonic surgical instrument or RF electrosurgical instrument), and take any other such action described above.

First 5202, the hospital staff members retrieve the patient's EMR from the hospital's EMR database. Based on select patient data in the EMR, the surgical hub 5104 determines that the procedure to be performed is a thoracic procedure.

Second 5204, the staff members scan the incoming medical supplies for the procedure. The surgical hub 5104 cross-references the scanned supplies with a list of supplies that are utilized in various types of procedures and confirms that the mix of supplies corresponds to a thoracic procedure. Further, the surgical hub 5104 is also able to determine that the procedure is not a wedge procedure (because the incoming supplies either lack certain supplies that are necessary for a thoracic wedge procedure or do not otherwise correspond to a thoracic wedge procedure).

Third 5206, the medical personnel scan the patient band via a scanner 5128 that is communicably connected to the surgical hub 5104. The surgical hub 5104 can then confirm the patient's identity based on the scanned data.

Fourth 5208, the medical staff turns on the auxiliary equipment. The auxiliary equipment being utilized can vary according to the type of surgical procedure and the techniques to be used by the surgeon, but in this illustrative case they include a smoke evacuator, insufflator, and medical imaging device. When activated, the auxiliary equipment that are modular devices 5102 can automatically pair with the surgical hub 5104 that is located within a particular vicinity of the modular devices 5102 as part of their initialization process. The surgical hub 5104 can then derive contextual information about the surgical procedure by detecting the types of modular devices 5102 that pair with it during this pre-operative or initialization phase. In this particular example, the surgical hub 5104 determines that the surgical procedure is a VATS procedure based on this particular combination of paired modular devices 5102. Based on the combination of the data from the patient's EMR, the list of medical supplies to be used in the procedure, and the type of modular devices 5102 that connect to the hub, the surgical hub 5104 can generally infer the specific procedure that the surgical team will be performing. Once the surgical hub 5104 knows what specific procedure is being performed, the surgical hub 5104 can then retrieve the steps of that procedure from a memory or from the cloud and then cross-reference the data it subsequently receives from the connected data sources 5126 (e.g., modular devices 5102 and patient monitoring devices 5124) to infer what step of the surgical procedure the surgical team is performing.

Fifth 5210, the staff members attach the EKG electrodes and other patient monitoring devices 5124 to the patient. The EKG electrodes and other patient monitoring devices 5124 are able to pair with the surgical hub 5104. As the surgical hub 5104 begins receiving data from the patient monitoring devices 5124, the surgical hub 5104 thus confirms that the patient is in the operating theater.

Sixth 5212, the medical personnel induce anesthesia in the patient. The surgical hub 5104 can infer that the patient is under anesthesia based on data from the modular devices 5102 and/or patient monitoring devices 5124, including EKG data, blood pressure data, ventilator data, or combinations. Upon completion of the sixth step 5212, the pre-operative portion of the lung segmentectomy procedure is completed and the operative portion begins.

Seventh 5214, the patient's lung that is being operated on is collapsed (while ventilation is switched to the contralateral lung). The surgical hub 5104 can infer from the ventilator data that the patient's lung has been collapsed. The surgical hub 5104 can infer that the operative portion of the procedure has commenced as it can compare the detection of the patient's lung collapsing to the expected steps of the procedure (which can be accessed or retrieved previously) and thereby determine that collapsing the lung is the first operative step in this particular procedure.

Eighth 5216, the medical imaging device 5108 (e.g., a scope) is inserted and video from the medical imaging device is initiated. The surgical hub 5104 receives the medical imaging device data (i.e., still image data or live streamed video in real time) through its connection to the medical imaging device. Upon receipt of the medical imaging device data, the surgical hub 5104 can determine that the laparoscopic portion of the surgical procedure has commenced. Further, the surgical hub 5104 can determine that the particular procedure being performed is a segmentectomy, as opposed to a lobectomy (note that a wedge procedure has already been discounted by the surgical hub 5104 based on data received at the second step 5204 of the procedure). The data from the medical imaging device 124 (FIG. 2) can be utilized to determine contextual information regarding the type of procedure being performed in a number of different ways, including by determining the angle at which the medical imaging device is oriented with respect to the visualization of the patient's anatomy, monitoring the number or medical imaging devices being utilized (i.e., that are activated and paired with the surgical hub 5104), and monitoring the types of visualization devices utilized.

For example, one technique for performing a VATS lobectomy places the camera in the lower anterior corner of the patient's chest cavity above the diaphragm, whereas one technique for performing a VATS segmentectomy places the camera in an anterior intercostal position relative to the segmental fissure. Using pattern recognition or machine learning techniques, for example, the situational awareness system can be trained to recognize the positioning of the medical imaging device according to the visualization of the patient's anatomy. As another example, one technique for performing a VATS lobectomy utilizes a single medical imaging device, whereas another technique for performing a VATS segmentectomy utilizes multiple cameras. As yet another example, one technique for performing a VATS segmentectomy utilizes an infrared light source (which can be communicably coupled to the surgical hub as part of the visualization system) to visualize the segmental fissure, which is not utilized in a VATS lobectomy. By tracking any or all of this data from the medical imaging device 5108, the surgical hub 5104 can thereby determine the specific type of surgical procedure being performed and/or the technique being used for a particular type of surgical procedure.

Ninth 5218, the surgical team begins the dissection step of the procedure. The surgical hub 5104 can infer that the surgeon is in the process of dissecting to mobilize the patient's lung because it receives data from the RF or ultrasonic generator indicating that an energy instrument is being fired. The surgical hub 5104 can cross-reference the received data with the retrieved steps of the surgical procedure to determine that an energy instrument being fired at this point in the process (i.e., after the completion of the previously discussed steps of the procedure) corresponds to the dissection step.

Tenth 5220, the surgical team proceeds to the ligation step of the procedure. The surgical hub 5104 can infer that the surgeon is ligating arteries and veins because it receives data from the surgical stapling and cutting instrument indicating that the instrument is being fired. Similarly to the prior step, the surgical hub 5104 can derive this inference by cross-referencing the receipt of data from the surgical stapling and cutting instrument with the retrieved steps in the process.

Eleventh 5222, the segmentectomy portion of the procedure is performed. The surgical hub 5104 infers that the surgeon is transecting the parenchyma based on data from the surgical instrument, including data from a staple cartridge. The cartridge data may correspond to size or type of staple being fired by the instrument. The cartridge data can indicate the type of tissue being stapled and/or transected for different types of staples utilized in different types of tissues. The type of staple being fired is utilized for parenchyma or other tissue types to allow the surgical hub 5104 to infer that the segmentectomy procedure is being performed.

Twelfth 5224, the node dissection step is then performed. The surgical hub 5104 can infer that the surgical team is dissecting the node and performing a leak test based on data received from the generator indicating that an RF or ultrasonic instrument is being fired. For this particular procedure, an RF or ultrasonic instrument being utilized after parenchyma was transected corresponds to the node dissection step, which allows the surgical hub 5104 to make this inference. It should be noted that surgeons regularly switch back and forth between surgical stapling/cutting instruments and surgical energy (i.e., RF or ultrasonic) instruments depending upon the particular step in the procedure because different instruments are better adapted for particular tasks. Therefore, the particular sequence in which the stapling/cutting instruments and surgical energy instruments are used can indicate what step of the procedure the surgeon is performing. Upon completion of the twelfth step 5224, the incisions and closed up and the post-operative portion of the procedure begins.

Thirteenth 5226, the patient's anesthesia is reversed. The surgical hub 5104 can infer that the patient is emerging from the anesthesia based on the ventilator data (i.e., the patient's breathing rate begins increasing), for example.

Lastly, fourteenth 5228, the medical personnel remove the various patient monitoring devices 5124 from the patient. The surgical hub 5104 can thus infer that the patient is being transferred to a recovery room when the hub loses EKG, BP, and other data from the patient monitoring devices 5124. The surgical hub 5104 can determine or infer when each step of a given surgical procedure is taking place according to data received from the various data sources 5126 that are communicably coupled to the surgical hub 5104.

In addition to utilizing the patient data from EMR database(s) to infer the type of surgical procedure that is to be performed, as illustrated in the first step 5202 of the timeline 5200 depicted in FIG. 11, the patient data can also be utilized by a situationally aware surgical hub 5104 to generate control adjustments for the paired modular devices 5102.

### Anticipation of Interactive Utilization of Common Data Overlays by Different Users

Having described a general implementation of the various surgical systems, surgical hubs, communication systems, augmentation systems, and augmented reality devices disclosed herein, such as surgical systems 1, 2, 50, 52, surgical hubs 6, 56, 5104, communication system 63, visualization system 8, augmentation system 83, imaging devices 24, 96 and AR devices 66, 84, the disclosure now turns to describe various other implantations of the systems, hubs, and devices. For the sake of brevity, various details and implementations of the systems, hubs, and devices described in the following sections, which are similar to the various systems, hubs, and devices described above, are not repeated herein. Any aspect of the systems, hubs, and devices described below can be brought into and/or be implemented by the above systems, hubs, and devices.

The operating room (OR) staff often includes a combination of members with different roles such as surgeons, anesthesiologists, nurse anesthetists, surgical technicians, residents, physician's assistants, etc. Throughout the course of a surgical procedure, staff members with varying roles may rely on different information in order to make decisions. Thus, if a staff member does not receive relevant information related to their role, it can result in critical errors related to the procedure.

To illustrate the importance of communicating relevant information to various OR staff members based on their role, two exemplary situations are provided below. The first exemplary situation relates to an abdominal perineal resection procedure performed by a surgical team and an anesthesiology team. During the procedure, the patient experienced some blood loss and the patient's surgical team was informed about the anesthesiology team's plan to transfuse the patient with a third unit of packed red blood cells. The surgical team members looked up in acknowledgement and continued surgery. Later on, a blood gas was drawn revealing the patient's hemoglobin (Hgb) level as 6.6 g/dL. Comparatively, at the start of the procedure, the patient's Hgb level was measured at 14.8 g/dL. When the surgical team was informed of the former Hgb measurement of 6.6 g/dL, the surgical team indicated that more bleeding should be expected. However, the surgical team did not indicate why they expected more bleeding. On three separate occasions, the attending anesthesiologist asked the surgical team for updates on the patient's surgical situation because the anesthesiology team was not able to keep up with the patient's blood, fluid, and resuscitation demands. Specifically, the amount and source of blood loss (e.g., arterial vs. venous vs. oozing), as well as the surgical team's plan to continue dissection despite continued bleeding, was not clearly communicated to the anesthesiology team. The patient's hemodynamics continued to worsen. As a result, the anesthesiology team initiated a massive transfusion protocol, a transesophageal electrocardiogram, and a rapid infuser. Additional anesthesia personnel were also called to assist. At this point, the surgical team finally disclosed to the anesthesiology team that the patient's iliac vein was nicked earlier in the surgery. Thus, as a result of the lack of real-time communication of relevant information from the surgical team, the anesthesiology team had dramatically underestimated how much blood loss the patient would suffer.

The second exemplary situation relates to an elective laparoscopic left nephrectomy procedure performed on a 43-year-old woman to remove a renal cell carcinoma. The OR staff included a surgical team lead by a head surgeon and an anesthesiology team lead by a head anesthesiologist. Both the head surgeon and head anesthesiologist agreed that the patient's risk for perioperative complications was low. The procedure proceeded as expected and the patient woke from anesthesia in stable condition. However, a few hours later, the patient developed a tense, distended abdomen. Further, the patient showed an elevated heart rate and low blood pressure. The head surgeon evaluated the patient and believed that the patient was experiencing internal bleeding. Thus, the head surgeon returned the patient to the OR.

Back in the OR, a massive transfusion of blood products was initiated. After induction of anesthesia, the patient's blood pressure dropped significantly. The anesthesiology team administered medications to try and improve the patient's blood pressure. Further, the surgical team opened the patient's abdomen and confirmed the presence of significant internal bleeding. At this point, a vascular surgeon was called to the room and the surgical staff worked to attempt to control the patent's bleeding. The head anesthesiologist continued to deliver blood products and escalated doses of blood pressure-augmenting medications. However, ST elevations on the patient's electrocardiogram indicated cardiac compromise. The head anesthesiologist communicated this information to surgical team members. The surgical team continued to try to identify the source of bleeding.

Eventually, the surgical team identified patient's inferior vena cava (IVC) as the source of the bleeding. However, the patient was experiencing cardiac arrest. In response, the surgical team stopped operating as the anesthesiology team attempted to resuscitate the patient. After 10 minutes of resuscitation attempts during which more than 100 units of blood products were administered, spontaneous circulation was achieved. Although the surgical team resumed operation, the head anesthesiologist was concerned about the patient's neurological status. Specifically, anesthetics had not been administered for several minutes suggesting the patient suffered a brain injury resulting from low blood pressure. The patient again began to experience cardiac arrest. Although the anesthesiology team attempted resuscitation, they eventually stopped because they believe the patient was moribund. The surgical team, however, requested that the anesthesiology team proceed with resuscitation as they attempted to control the patients IVC bleeding.

As can be appreciated from these two exemplary situations, the various surgical staff members can require different information, depending on their role, to proceed with decision making. For example, members of the anesthesiology team may need to monitor both neurosurgical-related and cardiac-related information. The neurosurgical-related information can include, for example, adequacy of oxygenation, deviation from expected hemodynamic parameters, blood loss, difficulties with hemostasis, relevant medical history of the patient (e.g., patient risk level), level of consciousness, brainstem reflexes, gaze, muscle tone, responses to pain, and the presence of adventitious movements in the eyes, face, or limbs. The cardiac-related information can include, for example, echocardiograph data, volume status, electrolyte levels, presence of acidosis, relevant medical history of the patient (e.g., patient risk level), patient position (e.g., during renal cell tumor removal), information from the surgical team, and intraabdominal pressure resulting from insufflation (e.g., related to risk of hypertension, increased myocardial workload, reduction in venous return, decreased preload, reduced end-diastolic volume). Thus, there is a wide variety of information that an anesthesiology team member may need to access during a surgical procedure. Likewise, surgical team members, and other members of the OR staff can have similarly extensive streams of information, specific to their role, that they may need to rely on during a surgical procedure. Accordingly, there is a need for apparatuses, systems, and methods for selectively presenting information based on a plurality of data streams to multiple users of a surgical system.

In various aspects, apparatuses, systems, and methods for selectively presenting information based on a plurality of data streams to multiple users of a surgical system are disclosed herein. The apparatuses, systems, and methods include displaying interactive overlays to provide information customized for a specific user.

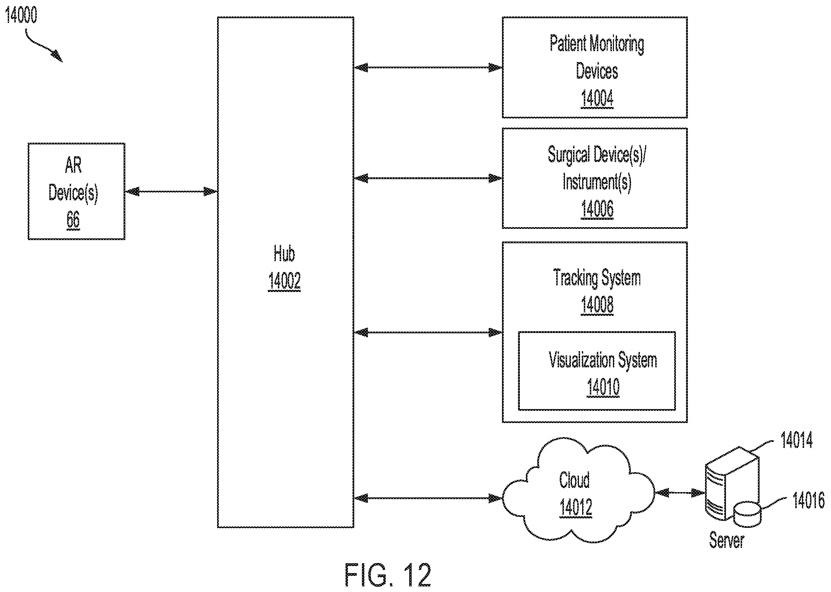

FIG. 12 depicts a surgical system 14000 configured to display interactive overlays for multiple users based on a plurality of data streams, according to several non-limiting aspects of this disclosure. The surgical system 14000 can include a surgical hub 14002 in communication with patient monitoring devices 14004, surgical devices/instruments 14006, tracking system 14008, and visualization system 14010. The surgical system 14000, surgical hub 14002, surgical devices/instruments 14006, and visualization system 14010 can be similar in many aspects, respectively, to any of the surgical systems, surgical hubs, surgical instruments/devices, and visualization systems described above, (e.g., surgical systems 1, 2, 50, 52; surgical hub 6, 56, 5104; device/instrument 21; visualization systems 8, 58). As explained above, surgical instruments may be communicatively connected to the surgical hub (e.g., device/instrument 21 can be connected to surgical hub 56 of FIG. 5). Thus, the surgical hub 14002 can be configured to receive instrument data from the surgical devices/instruments 14006 related to various sensed parameters and operational settings of the devices/instruments 14006. Based on the instrument data received by the surgical hub 14002, the surgical hub 14002 can determine operating parameters of the instrument. For example, based on instrument data received from the surgical devices/instruments 14006, the surgical hub 14002 can determine operating parameters such as speed, force, firing speed, firing force, activation status, power level, activation time, energy mode, and instrument settings.

Still referring to FIG. 12, the patient monitoring devices 14004 can be any type of device(s) configured to monitor aspects of a patent related to a surgical procedure. For example, referring again to the exemplary surgical situations discussed above, the patient monitoring devices 14004 can be configured to monitor aspects of the patient that may be used by a member of the anesthesiology team (e.g., oxygenation, hemodynamic parameters, blood loss, echocardiograph data, volume status, electrolyte levels, intraabdominal pressure resulting from insufflation, etc.). The surgical hub 14002 can be configured to receive patient monitoring data from the patient monitoring devices 14004.

The tracking system 14008 and/or the visualization system 14010 can be similar, in many aspects, to the tracking system 15006 and visualization system 15008 discussed in the U.S. Patent Application titled MIXING DIRECTLY VISUALIZED WITH RENDERED ELEMENTS TO DISPLAY BLENDED ELEMENTS AND ACTIONS HAPPENING ON-SCREEN AND OFF-SCREEN, Attorney Docket Number END9352USNP11 / 210120-11. The tracking system 14008 can be configured to track the location, position, motion, and/or other attributes of various objects within the OR based on one or more different types of tracking methods. For example, the tracking system 14008 and/or visualization system 1400 can utilize any combination of imaging devices (e.g., cameras, visual and/or non-visual image sensors, etc.), structured light sensors, LIDAR (Light Detection and Ranging) sensors, floor sensors, acoustic sensors, fiducial markers, user/device sensors, and GPS (global positioning system) sensors to track the position, location, and/or movement of objects in the OR. The objects tracked by the tracking system can include OR staff members, surgical devices/instruments 14006, a patient, AR devices 66, equipment, etc. In some aspects, the surgical hub 14002 can be configured to receive data from the tracking system 14008 and/or visualization system 14010 to determine the relative position of tracked objects, interactions between tracked objects, and/or the proximity of tracked objects to each other.

In various aspects, the surgical hub 14002 can be communicatively coupled to a cloud 14012 that can include a remote server 14014 having data storage 14016. The cloud 14012, remote server 14014, and data storage 14016 can be similar in many aspects, respectively, to any of the clouds, remote servers, and data storage devices described herein, (e.g., cloud 54, remote server 63, data storage 55 of FIG. 5). In some aspects, data storage 14016 can store information related to a patient undergoing a surgical procedure. The patient information can include, for example, information related to a patient's medical history (e.g., patient risk level). The surgical hub can be configured to retrieve data from data storage 14016.

Accordingly, in some aspects, the surgical hub 14002 can be configured to receive a plurality of data streams related to a surgical procedure. The plurality of data streams related to a surgical procedure can include any combination of data received from patient monitoring devices 14004, surgical devices/instruments 14006, tracking system 14008, visualization system 14010, and/or server 14014.

Still referring to FIG. 12, the surgical hub 14002 can be communicatively connected to AR devices 66. As explained above, the AR devices 66 can be similar, in many aspects, to the AR device 84 disclosed with respect to FIG. 10. The surgical hub 14002 can cause the AR devices 66 to display information based on any of the plurality of data streams received from the patient monitoring devices 14004, surgical devices/instruments 14006, tracking system 14008, visualization system 14010, and/or server 14014. For example, the surgical hub 14002 can be configured to cause an AR device 66 to display an interactive overlay including information related to monitored aspects of a patient, surgical instrument operating parameters, tracked objects (e.g., device interactions), various captured images of a surgical field, etc. Thus, a user (*e.g.,* an OR staff member) using the AR device 66 is able receive information related to a surgical procedure, in real time, based on at least some of the plurality of different data streams. The user can use this information to assist with decision making during the surgical procedure. Moreover, because of the real-time display of this information, the user may be able to more effectively make decisions that affect critical aspects of the surgical procure compared to other methods of communicating information in the OR.

In some aspects, the interactive overlays displayed by the AR device 66 can be customized based on the user who is using the AR device 66. Moreover, if multiple users are using different AR devices 66, the interactive overlays displayed by each of the different AR devices 66 can be customized based on each user. For example, as described in the exemplary surgical situations above, OR staff members can often include a surgical team and an anesthesiology team. The surgical team members and the anesthesiology team members may each be wearing AR devices 66. The surgical hub 14002 can be configured to cause the AR devices 66 worn by the surgical team to display a first interactive overlay customized based on the informational needs of the surgical team. The first interactive overlay can include information such as an operating parameter of a device that a surgical team member is using and/or patient information that is relevant to a step of a procedure that the surgical team is performing. Moreover, the surgical hub 14002 can be configured to cause the AR devices 66 worn by the anesthesiology team to display a second interactive overlay customized based on the informational needs of the anesthesiology team. The second interactive overlay can include information such as an operating parameter of a device that an anesthesiology team member is using and/or patient information relevant to a step of a procedure that the anesthesiology team is performing.

In some aspects, the control of a surgical device/instrument 14006 may transfer from a first user to a second user during a surgical procedure. For example, the first user may hand the surgical device/instrument 14006 to the second user. Prior to the handoff, the AR device 66 worn by the first user can display an overlay based on data related to the surgical device/instrument 14006. The tracking system 14008 can be configured to detect that the first user has transferred control of the surgical device/instrument 14006 to the second user. Based on data from the tracking system 14008 related to the detected transfer, the surgical hub 14002 can cause the interactive overlays displayed by the first user's AR device 66 and the second user's AR device 66 to update. For example, after the handoff, the AR device 66 worn by the second user can display an overlay based on the data related to the transferred surgical device/instrument 14006. Additionally, the AR device 66 worn by the first user may stop displaying the overlay based on the data related to the transferred surgical device/instrument 14006.

In some aspects, the informational needs of users may overlap. Accordingly, the overlays displayed by the various AR devices 66 of the surgical system 14000 can be based on at least some of the same information (*i.e*., can be based on at least some of the same data streams received by the surgical hub 14002).

In some aspects, the AR devices 66 of the surgical system 14000 can be linked to specific users by the surgical hub 14002. The term "linked," as used herein when referring to an AR device being linked to a specific user, can mean that the surgical hub 14002 has determined that the specific user is using the AR device. Linking a specific AR device 66 to a specific user can allow the surgical hub 14002 to cause the specific AR 66 to display customized overlays for the specific user.

In some aspects, the surgical hub 14002 can link an AR device 66 to a user based on information stored by the surgical hub 14002 and/or based on data stored by data storage 14016. In one aspect, data storage 14016 can be a data storage device of a hospital network or a manufacturer of the surgical system 14000. The data storage device 14016 can include information identifying OR staff members expected to be present for various types of procedures. Further, data storage 14016 may include information related to the hospital's scheduling system, such as which staff members are scheduled to be present during a procedure. Accordingly, the surgical hub 14002 can be configured to identify specific AR devices 66 that can be linked to the expected OR staff members. Thus, the surgical hub 14002 can cause the AR devices 66 to display overlays customized based on the roles of the expected OR staff members. For example, referring again to the second exemplary surgical situation described above, the data storage device 14016 can store information related to the OR staff members expected to perform the elective laparoscopic left nephrectomy procedure. The expected staff members can include a surgical team and an anesthesiology team. The surgical hub 14002 can identify specific AR devices 66 that should be worn by the staff members based on their respective roles.

In some aspects, the surgical hub 14002 can be configured to provide external notifications when the expected OR staff members change during a surgical procedure. In one aspect, these external notifications may be provided via an AR device or other type of display device used by a staff member in a different operating room or elsewhere in the hospital (*e.g*., surgical system 14000 can be similar to the surgical data network 51 if FIG. 4, with a modular communication hub 53 configured to connect modular devices located in one or more operating theaters/rooms of a healthcare facility). For example, referring again to the second exemplary surgical situation described above, a specialist, such as vascular surgeon, may be called into an OR based on specific events (*e.g*., unexpected uncontrollable bleeding) that occur during a surgical procedure. The surgical hub 14002 can be configured to detect the occurrence of the event (*e.g*., the surgical hub 14002 can be similar to the situationally aware surgical hub 5104 described above with respect to FIG. 11) and cause a notification to be derived to the relevant staff member, such as the vascular surgeon from the example above, indicating that the staff member is needed or may be needed for the procedure. In one aspect, the notifications may be bypassed. In another aspect, the notifications may be classified based on risk level and/or severity of the detected event. For example, depending on a risk level of the detected event, the surgical hub 14002 may cause an early warning notification to be delivered to an on-call specialist indicating that the specialist may be needed in the OR. In another aspect, the notification may enable a staff member who is not in the OR to view an image of the surgical field and/or other overlays that staff members who are present in the OR are viewing. For example, upon receiving a notification, an on call vascular surgeon who is not present in the OR, but who is wearing an AR device 66, may be able to cause the AR device 66 to display the same interactive overlay that is being displayed on an AR device 66 of a member of the surgical team who is performing the surgical procedure in the OR (*e.g*., allowing the vascular surgeon to view vitals, warnings, etc.).

In some aspects, the OR staff members may be able to customize the overlays that are displayed by their AR devices 66. For example, at the start of a procedure, the AR device 66 may display a menu with multiple functionality levels selectable by the user. In one aspect, the menu may include three functionality levels, wherein selecting the first level causes the display of an overlay related to overarching position of the staff member, wherein selecting the second level causes the display of perioperative data and/or information related to the patient history, and wherein selecting the third level causes the display of preferences and/or and or priorities of the user. The user may be able to adjust settings related to each of the selected levels.

In some aspects, the surgical hub 14002 can link an AR device 66 to a user based on data from the tracking system 14008. In one aspect, the tracking system 14008 can be configured to detect when a specific user picks up or otherwise takes control over a specific AR device. For example, as explained in more detail in the U.S. Patent Application titled MIXING DIRECTLY VISUALIZED WITH RENDERED ELEMENTS TO DISPLAY BLENDED ELEMENTS AND ACTIONS HAPPENING ON-SCREEN AND OFF-SCREEN, Attorney Docket Number END9352USNP11 / 210120-11, the user may be wearing a user sensor (*e.g*., a smart glove) that is tracked by the tracking system 14008. The tracking system 14008 can detect the proximity of the user sensor to an AR device 66, and based on this detected proximity, the surgical hub 14002 can link the AR device 66 to the user. In another aspect, surgical hub 14002 can be configured to cause the linked AR device 66 to automatically display an overlay based on proximity of the tracked user sensor to a surgical device/instrument 14006. For example, the proximity of the user sensor to the surgical device/instrument 14006 (*e.g.,* a surgical stapler) may cause the linked AR device 66 to display parameters of the surgical device/instrument 14006 (*e.g.,* a power level, a type of staple cartridge installed therein, etc.).

In some aspects, upon linking an AR device 66 to a user by the surgical hub 14002, the surgical hub 14002 may cause an update to the settings of and/or overlays displayed by the AR device 66. For example, the surgical hub 14002 may determine that an AR device 66 is linked to a user with a specific experience level (*e.g*., a resident vs. an experienced surgeon). The surgical hub 14002 may cause the AR device 66 to display different levels of information depending on the experience level of the user. In another aspect, the surgical hub 14002 can select the information to display on the AR device 66 based on prior uses of the AR device 66 by a particular user (*e.g*., based on machine learning, based on the experience of the user with the AR device 66). In another aspect, a user can override the automatic linking of an AR device 66 to the user. In yet another aspect, a user can to manually link an AR device to the user (*e.g*., using controls included on the AR device 66).

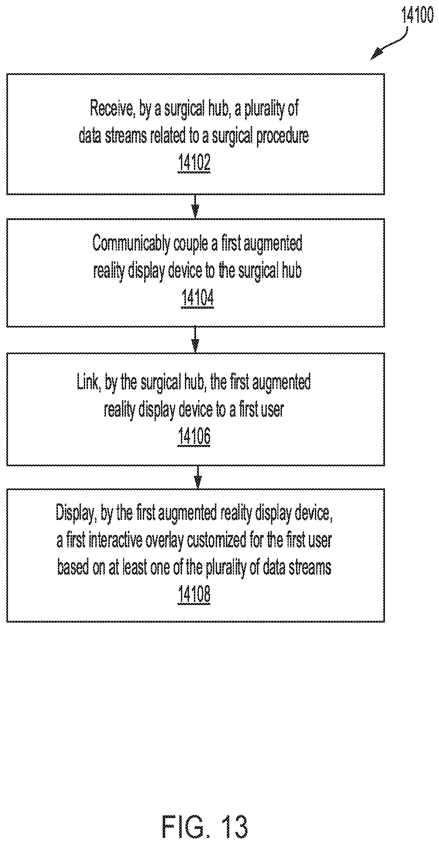

FIG. 13 illustrates a method 14100 of displaying interactive overlays for multiple users of a surgical system, according to several non-limiting aspects of this disclosure. The method 14100 may be practiced by any combination of the surgical systems, surgical hubs, tracking systems, visualization systems, patient monitoring devices, surgical devices/instruments, AR devices, any of the components thereof, and any other devices and systems disclosed herein, such as surgical systems 1, 2, 50, 52, 14000, surgical hubs 6, 56, 5104, 14002, tracking system 14008, visualization systems 8, 14010, patient monitoring devices 14004, surgical devices/instruments 14006, and AR devices 66, 84.

In accordance with the method 14100, a surgical hub can receive 14102 a plurality of data streams related to a surgical procedure. A first augmented reality display device can communicably couple 14104 to the surgical hub. The surgical hub can link 14106 the first augmented reality display device to a first user. The first augmented reality display device can display 14108 a first interactive overlay customized for the first user based on at least one of the plurality of data streams.

In accordance with one aspect of the method 14100, a second augmented reality display device can communicably couple to the surgical hub. Further, the surgical hub can link the second augmented reality display device to a second user. The second augmented reality display device can display a second interactive overlay customized for the second user based on at least one of the plurality of data streams.

In accordance with another aspect of the method 14100, the first augmented reality display device and the second augmented reality device can simultaneously display the first interactive overlay and the second interactive overlay. In another aspect, the first interactive overlay and the second interactive overlay can be based on the same data stream of the plurality of data streams.

In accordance with another aspect of the method 14100, the first augmented reality display device and the second augmented reality device can respectively display the first interactive overlay and the second interactive overlay differently for the first user and the second user based on the respective preferences of the first user and the second user.

In accordance with another aspect of the method 14100, the first augmented reality display device can display the first interactive overlay based on a first data stream related to a first surgical instrument when the first user is using the first surgical instrument and the second augmented reality display device can display the second interactive overlay based on a second data stream related to a second surgical instrument when the second user is using the second surgical instrument. In yet another aspect, the first augmented reality display device can update the first interactive overlay to be based on the second data stream when the first user is using the second surgical instrument. In one aspect, the plurality of data streams can include the first data stream and the second data stream.

### Decision Matrix for User Notification based on Risk-Level of Error and Surgeon Response

As explained in detail above, the communication of information can be critical during the performance of a surgical procedure. Accordingly, augmented reality systems utilizing data streams from multiple sources, such patient monitoring devices, surgical devices/instruments, tracking systems, and other data storage devices, may rely on the seamless integration of interconnected systems for the collection, interpretation, and communication of these data streams between devices and OR staff throughout a procedure. However, there may be situations where data is erroneously communicated and/or where communication of a data stream is interrupted or lost. Accordingly, there is a need for apparatuses, systems, and methods for detecting errors and/or data communication issues and determining the appropriate actions to implement when errors and/or data communication issues arise. Moreover, there is a need for apparatuses, systems, and methods that are able to implement an action based on varying levels of risk associated with the criticality of the detected errors and/or based on OR staff members' responses to prior warnings.

Apparatuses, systems, and methods for detecting errors and/or data communication issues and determining the appropriate actions to implement when the errors and/or data communication issues are detected are disclosed herein. In various aspects, the apparatuses, systems, and methods can implement a decision matrix based on the type of errors detected. The decision matrix can trigger various actions, such as user notification, a system override, and a device lockout, based on the varying degrees of risk associated with the detected errors. The decision matrix may also trigger various actions based on prior responses of the user to similar detected errors.

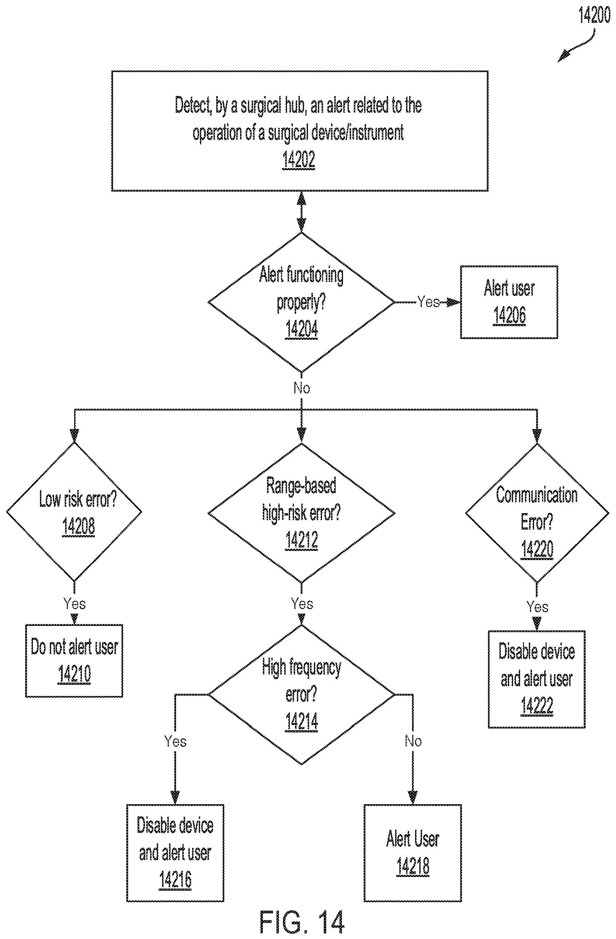

FIG. 14 illustrates a method 14200 for detecting a device-related error and determining actions to implement based on the detected error, according to several non-limiting aspects of this disclosure. The method 14200 may be practiced by any combination of the surgical systems, surgical hubs, tracking systems, visualization systems, patient monitoring devices, surgical devices/instruments, AR devices, any of the components thereof, and any other devices and systems disclosed herein, such as surgical systems 1, 2, 50, 52, 14000, surgical hubs 6, 56, 5104, 14002, tracking system 14008, visualization system 8, 14010, patient monitoring devices 14004, surgical devices/instruments 14006, and AR devices 66, 84.

Referring primarily to FIG. 14, and also to FIG. 12, in accordance with the method 14200, the surgical hub 14002 can detect 14202 an alert related to the operation of a surgical device/instrument 14006. For example, the device/instrument 14006 may indicate that an operating parameter of the instrument is outside of an expected and/or recommended operating range. Next, the surgical hub 14002 may determine 14204 the detected alert is functioning properly. For example, the surgical hub 14002 can determine 14204 that the operating parameter of device/instrument 14006 is in fact outside of the recommended operating range. Upon determining 14204 that the detected alert if functioning properly, the surgical hub 14002 can cause AR device 66 to display 14206 an alert (*e.g.*, an overlay) related to the detected error. For example, the AR device 66 may display an overlay instructing the user to take a specific action to address the detected error (*e.g*., waiting for a temperature of the instrument to return to the recommended operating range).

In another aspect of the method 14200, the surgical hub can determine 14204 that the detected alert is not functioning properly. Upon determining 14204 that the alert is not functioning properly, the surgical hub 14002 can determine 14208 whether the detected error is a low risk-level error. For example, the surgical hub 14002 may determine that the device/instrument 14006 is only generating erroneous data under a low risk condition (*e.g*., the instrument is only generating erroneously high temperature readings when the instrument is operating at low-temperature conditions). Upon determining 14208 that the detected error is a low risk-level error, the surgical hub 14002 can determine to not provide 14210 an alert to the user based on the detected error.

In another aspect, upon determining 14204 that the detected alert is not functioning properly, the surgical hub 14002 can determine 14212 whether the detected alert is a range-based high-risk error. For example, the surgical hub 14002 may determine that the device/instrument 14006 is generating erroneous data under a high-risk condition (*e.g*., the instrument is generating erroneously temperature readings when the instrument is operating at a high temperature, thereby causing a risk of overheating). Upon determining 14212 that the detected alert is a range-based high-risk error, the surgical hub 14002 can determine 14214 the frequency of the range-based high-risk error. For example, a range-based high-risk error may have a high frequency of error when the percentage of erroneous data generated by the device/instrument 14006 are above a predetermined threshold (*e.g*., 10%, 20%, 30%, 40%, 50%, 60%, 70%, 80%, or 90% of the erroneous readings generated by the instrument indicate that the operating parameter of the instrument is outside of the expected and/or recommended range). In various aspects, the frequency threshold may be determined 14214 based on a sampling rate of the data.

Upon determining 14214 that the range-based high-risk error is above a frequency threshold, the surgical hub 14002 can cause AR device 66 to display an alert (*e.g*., an overlay) related to the detected error and disable 14216 the surgical instrument/device 14006. For example, the surgical hub 14002 can cause the AR device 66 to display an overlay indicating that the instrument/device 14006 has been locked out due to errors related to the detected operating parameter. Conversely, upon determining 14214 that the range-based high-risk error is below a frequency threshold, the surgical hub 14002 can cause AR device 66 to display 14218 an alert (*e.g*., an overlay) related to the detected error. For example, the surgical hub 14002 can cause the AR device 66 to display an overlay instructing the user to verify the accuracy of the detected operating parameter via alternate means. In this aspect, the surgical hub 14002 may not lockout the surgical instrument/device 14006.

In another aspect of the method 14200, upon determining 14204 that the detected alert is not functioning properly, the surgical hub 14002 can determine 14220 whether the detected error is communication-related. A communication-related error can be caused by a loss of communication between the surgical hub 14002 and the surgical instrument 14006. In one aspect, upon determining 14220 that the detected alert is a communication-related error, the surgical hub 14002 can cause AR device 66 to display an alert (*e.g*., an overlay) related to the detected error. For example, the surgical hub 14002 can cause the AR device 66 to display an overlay indicating the loss of communication with the surgical instrument/device 14006. In another aspect, the surgical hub 14002 can disable 14222 the surgical instrument/device 14006, preventing further use of the instrument/device 14006.

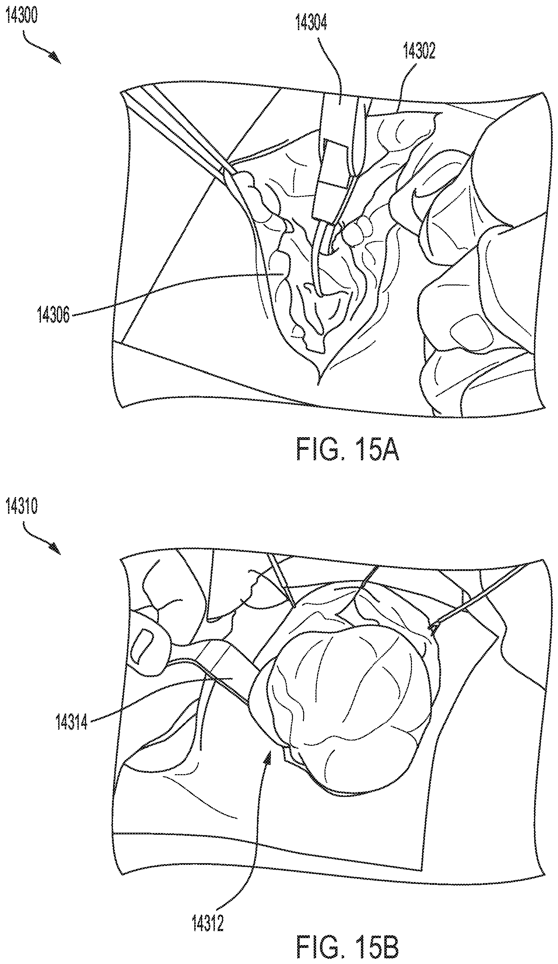

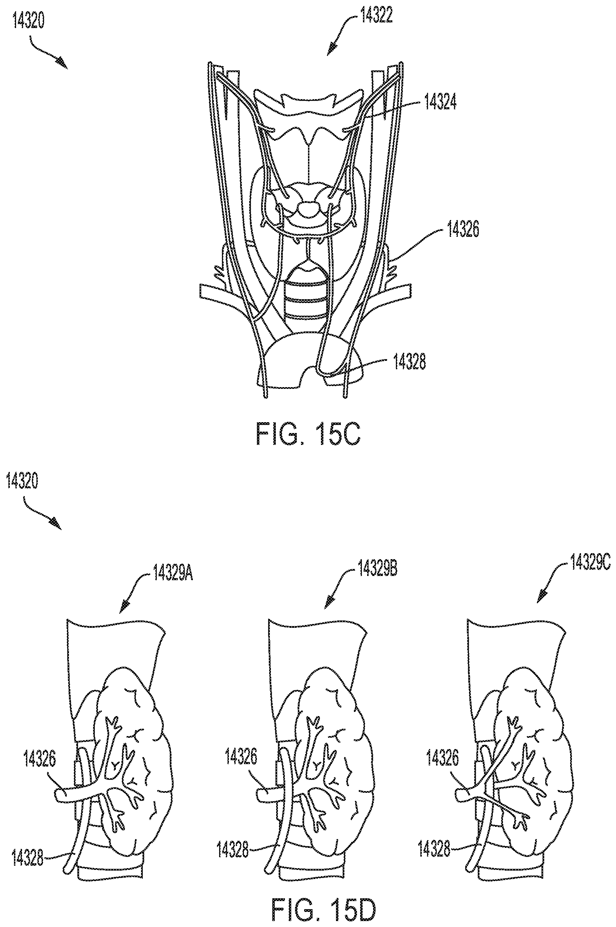

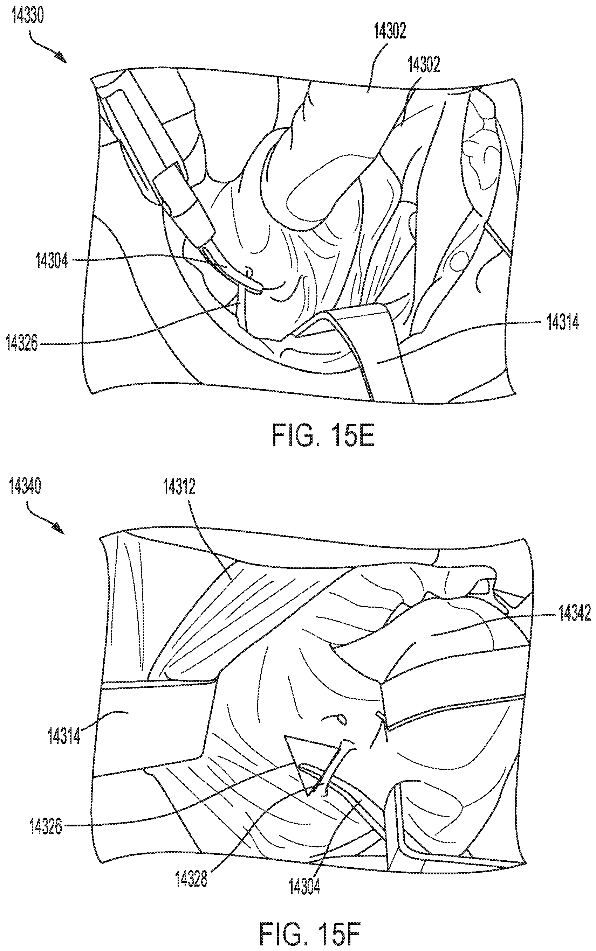

FIGs. 15A, 15B, 15C, 15D, 15E, and 15F illustrate an exemplary implementation of the method 14200 of FIG. 14 during a thyroidectomy procedure, according to several non-limiting aspects of this disclosure. Referring to FIG. 15A, a first step 14300 of the thyroidectomy procedure can include making an incision 14302 to gain access to underlying tissue 14306. The first step 14300 can also include using an ultrasonic energy device 14304 to dissect platysmal muscle in the underlying tissue 14306. At this point in the procedure, the blade of the ultrasonic energy device 14304 can begin to heat up from repeated use. Referring now to FIG. 15A and FIG. 12, the ultrasonic energy device 14304 can be a surgical instrument/device 14006 in communication with the surgical hub 14002. The surgical hub 14002 can receive data from the ultrasonic energy device 14304 related to the temperature of the blade.

Referring now to FIG. 15B, a second step 14310 of the thyroidectomy procedure can include further dissecting strap muscles 14312. This second step 14310 may be performed using a blunt device 14314 (shown) or the ultrasonic energy device 14304 (not shown). Use of the ultrasonic energy device 14304 can cause the blade to continue to raise in temperature.

Referring now to FIGs. 15C and 15D, a third step 14320 of the thyroidectomy procedure can include identifying critical structures such as the superior thyroid artery 14324, the inferior thyroid artery 14326, and the recurrent laryngeal nerve 14328. FIG. 15C illustrates a schematic of an exemplary anatomy 14322 of the superior thyroid artery 14324, the inferior thyroid artery 14326 and the recurrent laryngeal nerve 14328. FIG. 15D illustrates how anatomies 14329A, 14329B, and 14329C can vary with different configurations of the recurrent laryngeal nerve 14328 with respect to the inferior thyroid artery 14326. The third step 14320 can be an importation step of the thyroidectomy procedure because it is generally critical that the recurrent laryngeal nerve 14328 not be damaged.

Referring now to FIG. 15E, a fourth step 14330 thyroidectomy procedure can include dividing the inferior thyroid artery 14326 using the ultrasonic energy device 14304. However, as mentioned above, the temperature of the blade of the ultrasonic energy device 14304 may continue to increase in temperature as it is used during the procedure. If the blade temperature overheats, the recurrent laryngeal nerve 14328 may be damaged. For example, a growing degree of lateral thermal spread occurs as heat builds at the blade. Activation of the ultrasonic energy device 14304 in tight spaces near the laryngeal nerve 14328 can cause damage to the nerve 14328. As another example, between activations, the surgeon may want to use the ultrasonic energy device 14304 to grasp and manipulate tissue. Doing so with a hot blade can cause damage to the laryngeal nerve 14328. As yet another example, between activations, in tight spaces, slight movement of the ultrasonic energy device 14304 can cause contact with the laryngeal nerve 14328. An inexperienced user may not anticipate tissue effects caused by the ultrasonic energy device 14304 when the user is not actively applying energy. Thus, it can be important for the ultrasonic energy device 14304 to communicate accurate temperature readings to the surgical hub. Moreover, if there is an error related to the temperature readings, the surgical hub may need to respond appropriately to avoid damage to the laryngeal nerve 14328.

Referring still to FIG. 15E, and also to FIGs. 12 and 14, upon receiving a hot blade notification from the ultrasonic energy device 14304, the surgical hub 14002 may implement the method 14200 for detecting device-related errors and determining actions to implement based on the detected error. For example, the surgical hub 14002 can detect 14202 a hot blade notification. If the surgical hub 14002 determines 14204 the detected alert is functioning properly, the surgical hub 14002 can cause AR device 66 to display an alert 14206 (*e.g*., an overlay) instructing the user to pause in order to cool off the blade of the ultrasonic energy device 14304. Once cool, the AR device 66 may indicate that the user can proceed without risk of damaging the laryngeal nerve 14328 because of a hot blade.

However, the surgical hub 14002 may determine 14208 the hot blade notification is a low risk-level error. For example, the surgical hub 14002 may determine that the ultrasonic energy device 14304 is only generating the hot blade notification when the blade is not actually overheating. In this case, the surgical hub 14002 can determine that no alert will be provided 14210 to the user.

Referring still to FIG. 15D, and also to FIGs. 12 and 14, the surgical hub 14002 may determine 14212 that the hot blade notification is a range-based high-risk error. For example, ultrasonic energy device 14304 may be generating abnormally high temperature readings. The surgical hub 14002 can determine 14214 whether to disable 14216 the ultrasonic energy device 14304 and alert the user or only alert 14218 the user based on the frequency of the high temperature readings.

Referring still to FIG. 15D, and also to FIGs. 12 and 14, the surgical hub 14002 may determine 14220 that the hot blade notification is based on a loss of communication with the ultrasonic energy device 14304. In this case, the surgical hub 14002 can 14222 disable (*e.g*., lockout) ultrasonic energy device 14304 and the cause AR device 66 to display an alert notifying the user of the communication loss. The lockout may be temporary, giving the blade of the ultrasonic energy device 14304 time to cool. Moreover, the duration of the temporary lockout may be based on the time it takes for the blade to cool.

Referring now to FIG. 15F, after a hot blade notification has been addressed according to method 14200, the final steps 14340 of the thyroidectomy procedure can include rotating the thyroid lobe 14342 to gain access to the ligament of Berry and associated minor vasculature. Further, the thyroid may be removed, the wound may be irrigated, the strap muscles 14312 may be closed, and the skin may be closed.

### Deployment Of Overlays And Managing Power Requirements

As explained in detail above, the communication of information can be critical during the performance of a surgical procedure. In some aspects, information may be communicated to users (*e.g*., OR staff members) via intraoperative displayed by wearable display devices (*e.g*., AR device 66, 84). In some aspects, the AR devices may be powered using battery power with a limited battery life. Thus, there may be a risk that an AR display device will lose power and/or have low power during a surgical procedure. Accordingly, there is a need for apparatuses, systems, and methods for managing power of AR devices.

In various aspects, apparatuses, systems, and methods for managing power of AR devices are disclosed herein. In some aspects, the power of AR devices (*e.g*., AR device 66, 84) may be managed by ensuring that a second AR device is available for a user in case a first AR device used by the user loses power. Referring again to FIG. 12, the surgical system 14000 can include a surgical hub 14002 in communication with AR devices 66. In one aspect, each AR device 66 in communication with the surgical hub 14002 can have the same capabilities (*i.e*., the same functionality). Moreover, as explained above, various AR devices 66 can display customized information based on the user that the device is linked to. Thus, in the case of a loss of power in a first AR device 66 worn by a first user (*e.g.*, an AR device 66 worn by a surgeon), a second AR device 66 (*e.g.,* a spare AR device 66, an AR device 66 worn by a different member of the OR staff) can be linked with the first user. For example, the first user can be associated with a first user profile implemented by AR devices 66. The user profile can include various setting and preferences of the first user. Upon the first AR device 66 losing power, the second AR device 66 can be linked to the first user, causing the second AR device to implement the profile of the first user. In some aspects, the linking of the second AR device 66 to the first user can be executed by the surgical hub 14002 using the various techniques described herein. In another, aspect the linking of the second AR device 66 to the first user may be executed by selecting the first user's profile on the second AR device 66 (*e.g*., via ID scan).

In some aspects, one or more of the AR devices 66 can display notifications indicating the remaining power available for the various AR devices 66 worn by OR staff. For example, a circulating nurse may receive a notification indicating that an AR device 66 linked to a surgeon is at low power. As another example, a circulating nurse may receive a notification indicating the power level of all of the AR devices 66 linked to OR staff members. In one aspect, the notifications may be listed based on a priority level of each of the OR staff members (*e.g*., ranked based on which OR staff members have the lowest power lever, ranked based on which OR staff members are most critical to the performing the surgical procedure, etc.). In another aspect, the notification may include an estimated time of the battery life remaining on the AR device(s) 66. Accordingly, the circulating nurse can identify which OR staff member may need a new AR device 66 based on the power level notifications and prepare to provide the OR staff member(s) with replacement AR devices 66.

In other aspects, the power of an AR device 66 may be managed by prioritizing functions executed by the AR device 66. In one aspect, the AR device 66 can be configured to have various levels of functionality. For example, an AR device 66 may be configured to have three levels of functionality, wherein level 1 functionality enables the display of information related to overarching position (*e.g*., full imaging and overlay functionality), wherein level 2 functionally enables the display of information related to perioperative data and/or patient history, and wherein level 3 functionality enables the display of information related to user preferences and/or priorities. Level 1 functionality can include level 2 and level 3 functionality. Further, level 2 functionality can include level 3 functionality.

In some aspects, the AR device 66 can be configured to allow a user to select the level of functionality of the AR device 66. For example, prior to the start of surgical procedure, a user may select level 1 functionality (*e.g*., full power mode). As the battery of the AR device 66 approaches a depletion, the AR device may be configured to adjust the functionality level, thereby conserving battery life. For example, the AR device 66 may be configured to enter a low power mode where only level 2 or level 3 functionality is enabled. In one aspect, in low power mode, the AR device 66 may only display standard vitals, emergency alerts, and/or displays related to level 2 and/or level 3 functionality. In another aspect, in low power mode, the user may no longer have the ability to use high-power-consuming functionality (e.g., swiping to view the AR displays of other users). Accordingly, the AR device 66 can be configured to mitigate situations were low and/or completely depleted power levels interrupt the delivery of information to the user of the device.

### Multi-Level Pairing Methods To Ensure Device/System Security

Cyber security is often a concern in cases where various devices are connected wirelessly to a system. As explained in detail above, the various surgical systems described herein can include various smart devices (*e.g*., smart surgical instruments, patient monitoring devices, tracking devices, AR devices, etc.) that are wireless connected to a surgical hub. Thus, there is an opportunity for unauthorized devices to attempt to exploit the wirelessly capabilities of the surgical system. Moreover, the unauthorized communication between corrupt devices and various components of the surgical system could lead incorrect data being presented to OR staff members. If an OR staff member relies on this incorrect data, it could ultimately cause the OR staff member to make an incorrect decision during a surgical procedure. Accordingly, there is a need for apparatuses, systems, and devices for ensuring the secure pairing of various smart devices of the surgical system. Moreover, smart devices may be compromised and/or corrupted after the initial paring to the surgical system. Therefore, there is also a need for apparatuses, systems, and methods for verifying the secure and/or authenticated connection to paired smart devices.

Apparatuses, systems, and methods for ensuring the secure wireless paring of smart devices to a surgical system are disclosed herein. The smart devices discussed below can include any device disclosed herein that may be configured to wirelessly communicate with a surgical system (*e.g*., surgical instruments/devices 14006, AR devices 66, 84, etc.). Referring again to FIG. 12, a surgical system 14000 can include a surgical hub 14002, a tracking system 14008, a cloud 14012 including a server 14014, surgical devices/instruments 14006, and AR devices 66. In some aspects, and least some of the surgical devices/instruments 14006 and/or the AR devices 66 (sometimes collectively referred to herein as devices 14006, 66) can be configured to wirelessly pair with the surgical hub 14002. The surgical hub 14002 can be configured to classify the security level of the connection to the paired of a device 14006, 66 based on the technique that is used to pair the device 14006, 66. In one aspect, the classified security levels can include a high security level, a medium security level, and a low security level, with the high security level being more secure than the medium security level and the medium security level being more secure than the low security level.

In various aspects, a device 14006, 66 can be semi-automatically paired at an inventory level. Inventory level pairing can include the tracking system 14008 recognizing a device 14006, 66 as the OR is being populated (*i.e*., stocked) in preparation for a surgical procedure. For example, various imaging devices and/or other tracking techniques employed by the tracking system 14008 may automatically detect a device 14006, 66 as it enters the OR. The surgical hub 14002 may identify the device 14006, 66 based on data from the tracking system 14008. The surgical hub 14002 can be configured to cross reference the identified device 14006, 66 with data stored by the server 14014 (*e.g.,* a hospital network server, a device manufacturer server) to retrieve a MAC (media access control) address associated with the identified device 14006, 66. Based on the MAC address, the surgical hub 14002 may begin to actively search for the identified device 14006, 66 for wireless pairing. In one aspect, as the device 14006, 66 is powered on (*e.g*., OR staff inserts a battery into the device 14006, 66), the surgical hub 14002 can automatically pair the device. In this aspect, the surgical hub 14002 may identify the pairing of the device 14006, 66 as a high security level pairing. In another aspect, OR staff may manually pair the device 14006, 66 to the surgical hub (*e.g*., using controls / buttons included on the device 14006, 66). In this aspect, the surgical hub 14002 may identify the pairing of the device 14006, 66 as a medium security level pairing.

In various aspects, a device 14006, 66 can be semi-automatically paired at a packaging level. Packaging level pairing can include scanning, by the surgical hub 14002 or a component thereof, a QR (quick response) code included on the packaging of a device 14006, 66. For example, OR staff may bring the device 14006, 66 into the OR in preparation for a surgical procedure. The device 14006, 66 may still be in its packaging from the manufacture (*e.g*., Tyvek packaging). The packaging can include a QR code to identify the device. In one aspect, the OR staff may scan the QR code using an imaging device associated with the surgical hub 14002. In another aspect, an imaging device of the tracking system 14008 may automatically scan the QR code. The surgical system 14002 can be configured to identify the device 14006, 66 based on the scanned QR code. Thus, the surgical hub 14002 may begin to actively search for the identified device 14006, 66 for wireless pairing. In one aspect, as the device 14006, 66 is powered on (*e.g*., OR staff inserts a battery into the device 14006, 66), the surgical hub 14002 can automatically pair the device. In this aspect, the surgical hub 14002 may identify the pairing of the device 14006, 66 as a high security level pairing. In another aspect, OR staff may manually pair the device 14006, 66 to the surgical hub (*e.g*., using controls / buttons included on the device 14006, 66). In this aspect, the surgical hub 14002 may identify the pairing of the device 14006, 66 as a medium security level pairing.

In various aspects, a device 14006, 66 can be semi-automatically paired at a device level. Semi-automatic device level pairing can include scanning, by the surgical hub 14002 or a component thereof, a QR (quick response) code included on the device 14006, 66. For example, OR staff may bring the device 14006, 66 into the OR in preparation for a surgical procedure and remove it from its packaging. In one aspect, the OR staff may scan the QR code using an imaging device associated with the surgical hub 14002. In another aspect, an imaging device of the tracking system 14008 may automatically scan the QR code. The surgical system 14002 can be configured to identify the device 14006, 66 based on the scanned QR code. Thus, the surgical hub 14002 may begin to actively search for the identified device 14006, 66 for wireless pairing. In one aspect, as the device 14006, 66 is powered on (*e.g*., OR staff inserts a battery into the device 14006, 66), the surgical hub 14002 can automatically pair the device. In this aspect, the surgical hub 14002 may identify the pairing of the device 14006, 66 as a high security level pairing. In another aspect, OR staff may manually pair the device 14006, 66 to the surgical hub (*e.g*., using controls / buttons included on the device 14006, 66). In this aspect, the surgical hub 14002 may identify the pairing of the device 14006, 66 as a medium security level pairing.

In various aspects, a device 14006, 66 can be manually paired at a device level. Manual device level pairing can include an OR staff member initiating paring of a device 14006, 66 with the surgical hub 14002 using controls and/or buttons included on the device 14006, 66. For example, as OR staff brings the device 14006, 66 into the OR in preparation for a surgical procedure, the staff may insert a battery into the device 14006, 66 or otherwise power on the device 14006, 66. Further, staff may initiate pairing of the device 14006, 66 to the surgical hub 14002 by using the controls and/or buttons included on the device 14006, 66. Upon recognizing that the device 14006, 66 is attempting to pair, the surgical hub 14002 can be configured to cause the device 14006, 66 to ask the user to conform the paring. In this aspect, the surgical hub 14002 may identify the pairing of the device 14006, 66 as a low security level pairing.

Apparatuses, systems, and methods for verifying the secure and/or authenticated connection to paired smart devices (device 14006, 66) are also disclosed herein. Various techniques may be implemented to verify and/or authenticate connected devices 14006, 66 after pairing, such as verifying duplicate data streams, checksum checking, "dummy" signal checking, data error threshold checking, and pre-procedure checking of data streams.

In some aspects, the device 14006, 66 may be authenticated after initial pairing by verifying duplicate data streams sent by the device 14006, 66. For example, the device 14006, 66 can be configured to wirelessly transmit duplicate data streams (*e.g*., duplicate packets sent multiple times) to the surgical hub 14002. The surgical hub 14002 can be configured to cross check each of the received data streams to verify device 14006, 66 authenticity.

In some aspects, the device 14006, 66 may be authenticated based on "dummy" signal checking. A dummy signal may refer to a signal sent by the device 14006, 66 that the surgical hub 14002 can compare to an expected signal to verify the authenticity of the device 14006, 66. In one aspect, the dummy signal can be sent by the device 14006, 66 on a predefined cadence. The surgical hub 14002 can authenticate the device if it receives the dummy signal at the expected predefined cadence. In another aspect, the predefined cadence may be adjusted to ensure data authenticity. In yet another aspect, the surgical hub 14002 can be configured to authenticate the device 14006, 66 based on the timing of received dummy signals and/or data included in the dummy signals. The surgical hub 14002 may index signals received by various devices 14006, 66 so that the security of the various devices 14006, 66 can be verified based on specific time points. In yet another aspect, the dummy signal expected by the surgical hub 14002 can be based on a signal received by the surgical hub 14002 from the device 14006, 66 upon initial pairing. For example, a device 14006, 66 may be initially paired to the surgical hub 14002 based on semi-automatically paired at a packaging level, as described above. Upon pairing, the device 14006, 66 may send an initial dummy signal that is stored by the surgical hub 14002. The surgical hub 14002 can compare subsequent dummy signals received from the device 14006, 66 to the initial dummy signal to authenticate the device 14006, 66. In another aspect, the dummy signal can include data derived from manufacturing calibration of the device 14006, 66. The data derived from manufacturing calibration of the device 14006, 66 could be stored by the device (e.g., by the devices EEPROM). In another aspect, the QR code on the packaging of the device could include data related to the dummy signal.

In some aspects, the device 14006, 66 may be authenticated after initial pairing by monitoring a data error rate of the data received from the device 14006, 66 by the surgical hub 14002. For example, the surgical hub 14002 can be configured to identify a device 14006, 66 as corrupted if the error rate of data received from the device 14006, 66 exceeds a data error rate threshold. In one aspect, the data error rate threshold can be predefined (*e.g*., six sigma). In another aspect, the data error threshold can be determined based on a risk level associated with the device 14006, 66. For example, each type of device may have a different error rate threshold. As another example, the error rate threshold may be based on the type of procedure the device is performing (*e.g.,* based on wither critical structures are present in the surgical field, based on whether arteries are present in the surgical field, based on a location of the device, based on whether the device is performing a dissection and/or manipulating tissue). As yet another example, the error rate threshold may be based on a combination of the device type and the type or procedure the device is performing (*e.g*., an ultrasonic device performing a thyroidectomy may have a different error rate threshold compared to a device performing a PFS firing on a pulmonary artery or using a device being a grasper, etc.).

In some aspects, the device 14006, 66 may be authenticated by a pre-procedure check of the data stream received from the device 14006, 66 by the surgical hub 14002. For example, the device 14006, 66 may be an endo-cutter. Prior to the start of a surgical procedure, the surgical hub 14002 can require that the endo-cutter perform a test firing. Based on the data received by the surgical hub 14002 related to the test firing, the surgical hub 14002 can verify the authenticity of the connection between the endo-cutter (device 14006, 66) and the surgical hub 14002.

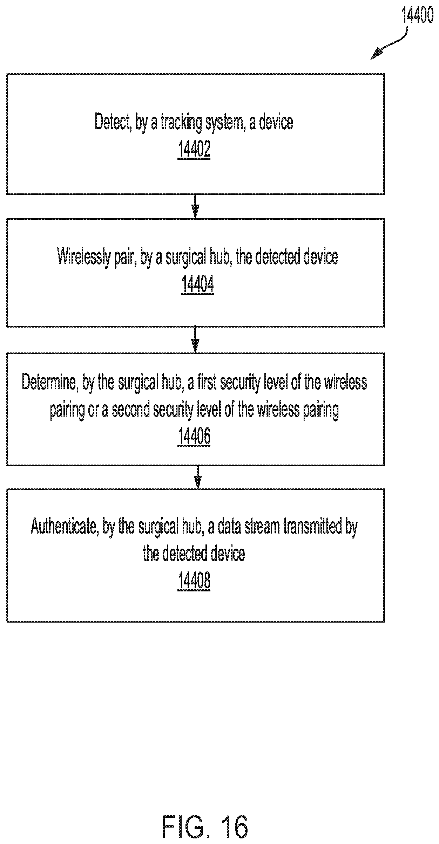

FIG. 16 illustrates a method 14400 for ensuring the secure wireless paring of smart devices to a surgical system, according to several non-limiting aspects of this disclosure. The method 14400 may be practiced by any combination of the surgical systems, surgical hubs, tracking systems, visualization systems, patient monitoring devices, surgical devices/instruments, AR devices, any of the components thereof, and any other devices and systems disclosed herein, such as surgical systems 1, 2, 50, 52, 14000, surgical hubs 6, 56, 5104, 14002, tracking system 14008, visualization system 8, 14010, patient monitoring devices 14004, surgical devices/instruments 14006, and AR devices 66, 84.

Referring primarily to FIG. 16, and also to FIG. 12, in accordance with the method 14400, a tracking system 14008 can detect 14402 a device 14006, 66. A surgical hub 14002 can wirelessly pair 14404 with the detected device 14006, 66. The surgical hub 14002 can determine 14406 a first or second security level of the wireless pairing, wherein the first security level is determined based on the detection 14002 of the device by the tracking system 14008 and an automatic wireless pairing of the detected device 14006, and wherein the second security level is determined on the detection 14002 of the device by the tracking system 14008 and a manual pairing of the detected device. Further, the surgical hub 14002 can authenticate 14408 a data stream transmitted by the detected device 14006, 66.

In accordance with one aspect of the method 14400, detecting 14002 the device 14006, 66 by the tracking system can include recognizing the device 14006, 66 as the OR is being populated. In another aspect of the method 14400, detecting 14002 the device 14006, 66 by the tracking system can include scanning a QR code included on packaging of the device 14006, 66. In yet another aspect of the method 14400, detecting 14002 the device 14006, 66 by the tracking system can include scanning a QR code included on the device 14006, 66.

### QR Code System To Confirm Data Transmission Accuracy And Latency In Connected OR Devices

As explained above, as devices and instruments gain the capability to connect within a digital OR ecosystem, concerns of data integrity (*e.g*., corrupt data being transmitted by devices) become more warranted. Moreover, in some aspects, the successful pairing of a device to a surgical hub does not preclude potentially faulty, corrupt, and/or delayed data being received by the surgical hub from the device. Simple approaches, such as checksums of data, may be employed to help ensure data authenticity and/or integrity. However, simple approaches can provide a false sense of data authenticity and integrity. For example, different permutations of bytes in a packet can generate the same checksum value. Thus, even though the checksum value appears to be authentic, the data received may actually be faulty, corrupt, etc. Accordingly, there is a need for apparatuses, systems, and methods for ensuring data authenticity and/or integrity after initial device pairing.

Apparatuses, systems, and methods for ensuring data authenticity and/or integrity after initial device pairing are disclosed herein. These apparatuses, systems, and methods can employ a two-part approach involving (i) the secure, initial pairing of a device to a surgical hub based on QR code and (ii) subsequent authentication of the pairing based on data transmitted to the surgical hub by the device at the initial pairing.

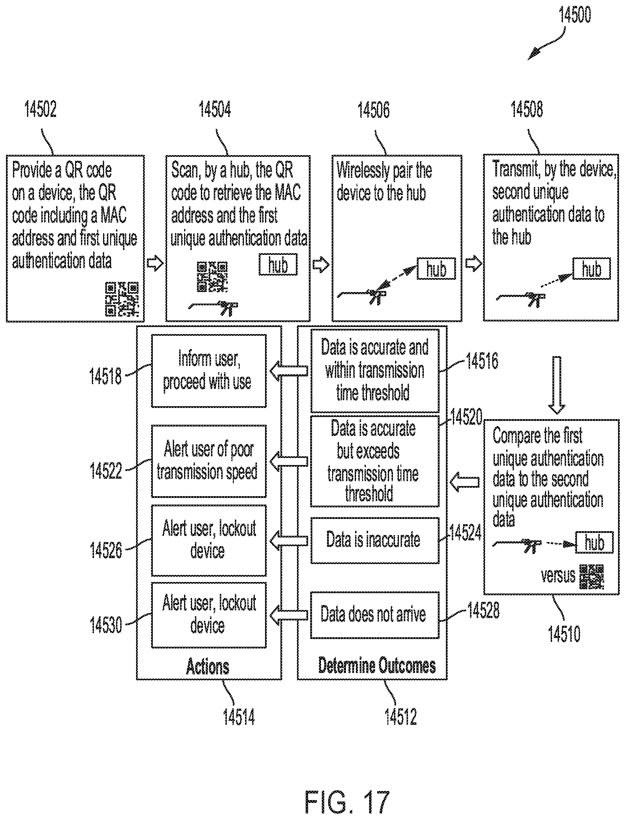

FIG. 17 illustrates a method 14500 for ensuring data authenticity and/or integrity after initial device pairing, according to several non-limiting aspects of this disclosure. The method 14500 may be practiced by any combination of the surgical systems, surgical hubs, tracking systems, visualization systems, patient monitoring devices, surgical devices/instruments, AR devices, any of the components thereof, and any other devices and systems disclosed herein, such as surgical systems 1, 2, 50, 52, 14000, surgical hubs 6, 56, 5104, 14002, tracking system 14008, visualization system 8, 14010, patient monitoring devices 14004, surgical devices/instruments 14006, and AR devices 66, 84.

Referring primarily to FIG. 17, and also to FIG. 12, in accordance with the method 14500, a QR code may be provided 14502 on a device 14006, 66 (*e.g.*, on the device packaging, on the device itself). The QR code can include information such as a media access control (MAC) address and first unique authentication data that can be used to uniquely identify the device 14006, 66. A component of the surgical system 14000, such as the surgical hub 14002 or the tracking system 14008 can scan 14504 the QR code to retrieve the MAC address and the first unique authentication data. Further, the surgical hub 14002 can wirelessly pair 14506 with the device 14006, 66 upon powering the device. After pairing 14506, the device 14006, 66 can transmit 14508 second unique authentication data to the surgical hub 14002. The surgical hub 14002 can compare 14510 the first unique authentication data to the second authentication data.

Still referring primarily to FIG. 17, and also to FIG. 12, the surgical hub 14002 can be configured to determine 14512 various comparison outcomes based on the comparison 14510. Further based on the determination 14512 of the comparison outcome, the surgical hub 14002 can cause various actions 14514. In one aspect, surgical hub can determine 14516 that the second unique authentication data is accurate and received within a transmission time threshold. Accordingly, the surgical hub 14002 can be configured to inform 14518 a user of the device 14006, 66 that the device 14006, 66 has been authenticated (*e.g*., via an overlay on AR device 66) and allow the user to proceed with using the device 14006, 66.

In another aspect, surgical hub can determine 14520 that the second unique authentication data is accurate but is not received within a transmission time threshold. Accordingly, the surgical hub 14002 can be configured to 14522 alert the user of the device's 14006, 66 poor transmission speed (*e.g*., via an overlay on AR device 66).

In another aspect, surgical hub can determine 14524 that the second unique authentication data is inaccurate. Accordingly, the surgical hub 14002 can be configured to alert 14526 the user of the device 14006, 66 that the device 14006, 66 has not been authenticated (*e.g*., via an overlay on AR device 66) and lockout the device 14006, 66.

In another aspect, surgical hub can determine 14528 that the second unique authentication data has not been received. Accordingly, the surgical hub 14002 can be configured to 14530 alert the user of the device 14006, 66 that the device 14006, 66 has not been authenticated (e.g., via an overlay on AR device 66) and lockout the device 14006, 66.

While several forms have been illustrated and described, it is not the intention of Applicant to restrict or limit the scope of the appended claims to such detail. Numerous modifications, variations, changes, substitutions, combinations, and equivalents to those forms may be implemented and will occur to those skilled in the art without departing from the scope of the present disclosure.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to control circuit, a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. A method for displaying interactive overlays for multiple users of a surgical system, the method comprising:
receiving, by a surgical hub, a plurality of data streams related to a surgical procedure;
communicably coupling a first augmented reality display device to a surgical hub;
linking, by the surgical hub, the first augmented reality display device to a first user;
displaying, by the first augmented reality display device, a first interactive overlay customized for the first user based on at least one of the plurality of data streams;
transmitting, by a first surgical instrument, a first surgical instrument data stream to the surgical hub, wherein in the first surgical instrument data stream is one of the plurality of data streams related to the surgical procedure;
detecting, by the surgical hub, an error related to the first surgical instrument data stream; and
causing, by the surgical hub, the first interactive data overlay to include an alert based on the detected error exceeding a risk level threshold.

2. The method of claim 1, further comprising:
communicably coupling a second augmented reality display device to the surgical hub;
linking, by the surgical hub, the second augmented reality display device to a second user; and
displaying, by the second augmented reality display device, a second interactive overlay customized for the second user based on at least one of the plurality of data streams.

3. The method of claim 2, further comprising simultaneously displaying the first interactive overlay by the first augmented reality display device and the second interactive overlay by the second augmented reality display device;
wherein the first interactive overlay and the second interactive overlay are based on the same data stream of the plurality of data streams.

4. The method of claim 3, further comprising displaying the first interactive overlay and the second interactive overlay differently for the first user and the second user based on the respective preferences of the first user and the second user.

5. The method of claim 2, further comprising:
displaying, by the first augmented reality display device, the first interactive overlay based on a first data stream related to a first surgical instrument when the first user is using the first surgical instrument;
displaying, by the second augmented reality display device, the second interactive overlay based on a second data stream related to a second surgical instrument when the second user is using the second surgical instrument; and
updating, by the first augmented reality display device, the first interactive overlay to be based on the second data stream when the first user is using the second surgical instrument;
wherein the plurality of data streams comprises the first data stream and the second data stream.

6. The method of claim 1, wherein the risk level threshold is based on a frequency of the detected error, the detected error exceeding expected range, a loss of communication with the first surgical instrument, or a combination thereof.

7. The method of claim 6, further comprising causing, by the surgical hub, a lockout of the first surgical instrument based on the detected error exceeding the risk level threshold.

8. The method of claim 1, further comprising:
automatically pairing, by the surgical hub, a surgical device to the surgical hub to achieve a first level of security or manually pairing, by a user, the surgical device to the surgical hub to achieve a second level of security; and
authenticating, by the surgical hub, a data stream transmitted between the surgical device and the surgical hub;
wherein the data stream is one of the plurality of data streams.

9. The method of claim 1, further comprising:
scanning, by the surgical hub, a quick response (QR) code of a surgical device to receive first device authentication data;
transmitting, by the surgical device, second device authentication data to the surgical hub, wherein the second device authentication data is one of the plurality of data streams;
comparing, by the surgical hub, the first device authentication data to the second device authentication data; and
displaying, by the first augmented reality display, a notification based on the comparison of the first device authentication data to the second device authentication data.

10. A surgical system for displaying interactive overlays for multiple users, the system comprising:
a surgical hub configured to receive a plurality of data streams related to a surgical procedure; and
a first augmented reality display device communicably coupled to the surgical hub, wherein the first augment reality display device is linked to a first user;
wherein the first augmented reality display device displays a first interactive overlay customized for the first user based on at least one of the plurality of data streams,
a first surgical instrument configured to transmit a first surgical instrument data stream to the surgical hub, wherein in the first surgical instrument data stream is one of the plurality of data streams related to the surgical procedure;
wherein the surgical hub is configured to detect an error related to the first surgical instrument data stream; and
wherein the surgical hub is configured to cause the first interactive data overlay to include a notification based on the detected error exceeding a risk level threshold.

11. The surgical system of claim 10, further comprising a second augmented reality display device communicably coupled to the surgical hub, wherein the second augment reality display device is linked to a second user;
wherein the second augmented reality display device displays a second interactive overlay customized for the second user based on at least one the plurality of data streams.

12. The surgical system of claim 11, wherein the first interactive overlay and the second interactive overlay are based on the same data stream of the plurality of data streams; and
wherein the first augmented reality display device and the second augmented reality display device display the first interactive overlay and the second interactive overlay simultaneously.

13. The surgical system of claim 12, wherein the first interactive overlay and the second interactive overlay are displayed differently for the first user and the second user based on the respective preferences of the first user and the second user.

14. The surgical system of claim 11, wherein the first interactive overlay is based on a first data stream related to a first surgical instrument when the first user is using the first surgical instrument;
wherein the second interactive overlay is based on a second data stream related to a second surgical instrument when the second user is using the second surgical instrument;
wherein the first interactive overlay updates to be based on the second data stream when the first user is using the second surgical instrument; and
wherein the plurality of data streams comprises the first data stream and the second data stream.

15. The surgical system of claim 10, wherein the risk level threshold is based on a frequency of the detected error, the detected error exceeding expected range, a loss of communication with the first surgical instrument, or a combination thereof.

16. The surgical system of claim 10, wherein the surgical hub is configured to cause a lockout of the first surgical instrument based on the detected error exceeding the risk level threshold.

17. The system of claim 10, further comprising a surgical device configured to be paired with the surgical hub;
wherein automatically pairing the a surgical device to the surgical hub achieves a first level of security;
wherein manually pairing the surgical device to the surgical hub to achieves a second level of security;
wherein the surgical hub is configured to authenticate a data stream transmitted between the surgical device and the surgical hub; and
wherein the data stream is one of the plurality of data streams.

18. The system claim 10, further comprising a surgical device comprising a quick response (QR) code, the surgical device configured to first device authentication data to the surgical hub;
wherein the surgical hub is configured to scan the QR code to receive second device authentication data;
wherein the surgical hub is configured to compare the first device authentication data and the second device authentication data;
wherein the first augmented reality display is configured to display a notification based on the first device authentication data and the second device authentication data; and
wherein the first device authentication data is one of the plurality of data streams.

## Patentansprüche

1. Verfahren zum Anzeigen interaktiver Overlays für mehrere Benutzer eines chirurgischen Systems, wobei das Verfahren umfasst:
Empfangen einer Vielzahl von Datenströmen im Zusammenhang mit einem chirurgischen Eingriff durch einen chirurgischen Knotenpunkt;
kommunikatives Koppeln eines ersten Augmented-Reality-Anzeigegeräts mit einem chirurgischen Knotenpunkt;
Verbinden des ersten Augmented-Reality-Anzeigegeräts mit einem ersten Benutzer durch den chirurgischen Knotenpunkt;
Anzeigen einer ersten interaktiven Überlagerung, die für den ersten Benutzer individuell angepasst ist, basierend auf mindestens einem der Vielzahl von Datenströmen durch das erste Augmented-Reality-Anzeigegerät;
Übertragen eines ersten chirurgischen Instrumentendatenstroms durch ein erstes chirurgisches Instrument an den chirurgischen Knotenpunkt, wobei der erste chirurgische Instrumentendatenstrom einer aus der Vielzahl von Datenströmen ist, die sich auf den chirurgischen Eingriff beziehen;
Erkennen eines Fehlers im Zusammenhang mit dem ersten Datenstrom chirurgischer Instrumente durch den chirurgischen Knotenpunkt; und
dadurch Veranlassen durch den chirurgischen Knotenpunkt, dass die erste interaktive Datenüberlagerung eine Warnung enthält, die auf dem erkannten Fehler basiert, der einen Risikoschwellenwert überschreitet.

2. Verfahren nach Anspruch 1, ferner umfassend:
kommunikatives Koppeln eines zweiten Augmented-Reality-Anzeigegeräts mit dem chirurgischen Knotenpunkt;
Verbinden des zweiten Augmented-Reality-Anzeigegeräts mit einem zweiten Benutzer durch den chirurgischen Knotenpunkt; und
Anzeigen einer zweiten interaktiven Überlagerung, die für den zweiten Benutzer individuell angepasst ist, basierend auf mindestens einem der Vielzahl von Datenströmen durch das zweite Augmented-Reality-Anzeigegerät.

3. Verfahren nach Anspruch 2, das ferner das gleichzeitige Anzeigen der ersten interaktiven Überlagerung durch das erste Augmented-Reality-Anzeigegerät und der zweiten interaktiven Überlagerung durch das zweite Augmented-Reality-Anzeigegerät umfasst;
wobei die erste interaktive Überlagerung und die zweite interaktive Überlagerung auf demselben Datenstrom der Vielzahl von Datenströmen basieren.

4. Verfahren nach Anspruch 3, das ferner das unterschiedliche Anzeigen der ersten interaktiven Überlagerung und der zweiten interaktiven Überlagerung für den ersten Benutzer und den zweiten Benutzer basierend auf den jeweiligen Präferenzen des ersten Benutzers und des zweiten Benutzers umfasst.

5. Verfahren nach Anspruch 2, ferner umfassend:
Anzeigen der ersten interaktiven Überlagerung durch das erste Augmented-Reality-Anzeigegerät basierend auf einem ersten Datenstrom, der sich auf ein erstes chirurgisches Instrument bezieht, wenn der erste Benutzer das erste chirurgische Instrument verwendet;
Anzeigen der zweiten interaktiven Überlagerung durch das zweite Augmented-Reality-Anzeigegerät basierend auf einem zweiten Datenstrom, der sich auf ein zweites chirurgisches Instrument bezieht, wenn der zweite Benutzer das zweite chirurgische Instrument verwendet; und
Aktualisieren der ersten interaktiven Überlagerung durch das erste Augmented-Reality-Anzeigegerät, sodass sie auf dem zweiten Datenstrom basiert, wenn der erste Benutzer das zweite chirurgische Instrument verwendet;
wobei die Vielzahl von Datenströmen den ersten Datenstrom und den zweiten Datenstrom umfasst.

6. Verfahren nach Anspruch 1, wobei der Risikoschwellenwert auf der Häufigkeit des erkannten Fehlers, der Überschreitung des erwarteten Bereichs durch den erkannten Fehler, einem Kommunikationsverlust mit dem ersten chirurgischen Instrument oder einer Kombination davon basiert.

7. Verfahren nach Anspruch 6, das ferner das Veranlassen einer Sperrung des ersten chirurgischen Instruments durch den chirurgischen Knotenpunkt umfasst, basierend auf dem erkannten Fehler, der den Risikoschwellenwert überschreitet.

8. Verfahren nach Anspruch 1, ferner umfassend:
automatisches Koppeln eines chirurgischen Geräts mit dem chirurgischen Knotenpunkt durch den chirurgischen Knotenpunkt, um eine erste Sicherheitsstufe zu erreichen, oder manuelles Koppeln des chirurgischen Geräts mit dem chirurgischen Knotenpunkt durch einen Benutzer, um eine zweite Sicherheitsstufe zu erreichen; und
Authentifizieren eines zwischen dem chirurgischen Gerät und dem chirurgischen Knotenpunkt übertragenen Datenstroms durch den chirurgischen Knotenpunkt;
wobei der Datenstrom einer aus der Vielzahl von Datenströmen ist.

9. Verfahren nach Anspruch 1, ferner umfassend:
Scannen eines Quick-Response-Codes (QR) eines chirurgischen Geräts durch den chirurgischen Knotenpunkt, um erste Geräteauthentifizierungsdaten zu erhalten;
Übertragen zweiter Geräteauthentifizierungsdaten durch das chirurgische Gerät an den chirurgischen Knotenpunkt, wobei die zweiten Geräteauthentifizierungsdaten einer der Vielzahl von Datenströmen sind;
Vergleichen der ersten Geräteauthentifizierungsdaten mit den zweiten Geräteauthentifizierungsdaten durch den chirurgischen Knotenpunkt; und
Anzeigen einer Benachrichtigung durch die erste Augmented-Reality-Anzeige basierend auf dem Vergleich der Authentifizierungsdaten des ersten Geräts mit den Authentifizierungsdaten des zweiten Geräts.

10. Chirurgisches System zur Anzeige interaktiver Overlays für mehrere Benutzer, wobei das System umfasst:
einen chirurgischen Knotenpunkt, der zum Empfangen einer Vielzahl von Datenströmen im Zusammenhang mit einem chirurgischen Eingriff konfiguriert ist; und
ein erstes Augmented-Reality-Anzeigegerät, das kommunikationsfähig mit dem chirurgischen Knotenpunkt verbunden ist, wobei das erste Augmented-Reality-Anzeigegerät mit einem ersten Benutzer verbunden ist;
wobei das erste Augmented-Reality-Anzeigegerät eine erste interaktive Einblendung anzeigt, die für den ersten Benutzer auf Grundlage von mindestens einem der Vielzahl von Datenströmen angepasst ist,
ein erstes chirurgisches Instrument, das dazu konfiguriert ist, einen ersten Datenstrom eines chirurgischen Instruments an den chirurgischen Knotenpunkt zu übertragen, wobei der erste Datenstrom eines chirurgischen Instruments einer aus der Vielzahl von Datenströmen ist, die sich auf den chirurgischen Eingriff beziehen;
wobei der chirurgische Knotenpunkt dazu konfiguriert ist, einen Fehler im Zusammenhang mit dem ersten chirurgischen Instrumentendatenstrom zu erkennen; und
wobei der chirurgische Knotenpunkt so konfiguriert ist, dass er die erste interaktive Datenüberlagerung veranlasst, eine Benachrichtigung einzuschließen, die auf dem erkannten Fehler basiert, der einen Risikoschwellenwert überschreitet.

11. Chirurgisches System nach Anspruch 10, das ferner ein zweites Augmented-Reality-Anzeigegerät umfasst, das kommunikationsfähig mit dem chirurgischen Knotenpunkt gekoppelt ist, wobei das zweite Augmented-Reality-Anzeigegerät mit einem zweiten Benutzer verbunden ist;
wobei das zweite Augmented-Reality-Anzeigegerät eine zweite interaktive Überlagerung anzeigt, die für den zweiten Benutzer individuell angepasst ist und auf mindestens einem der Vielzahl von Datenströmen basiert.

12. Chirurgisches System nach Anspruch 11, wobei die erste interaktive Überlagerung und die zweite interaktive Überlagerung auf demselben Datenstrom der Vielzahl von Datenströmen basieren; und
wobei das erste Augmented-Reality-Anzeigegerät und das zweite Augmented-Reality-Anzeigegerät die erste interaktive Überlagerung und die zweite interaktive Überlagerung gleichzeitig anzeigen.

13. Chirurgisches System nach Anspruch 12, wobei die erste interaktive Überlagerung und die zweite interaktive Überlagerung für den ersten Benutzer und den zweiten Benutzer basierend auf den jeweiligen Präferenzen des ersten Benutzers und des zweiten Benutzers unterschiedlich angezeigt werden.

14. Chirurgisches System nach Anspruch 11, wobei die erste interaktive Überlagerung auf einem ersten Datenstrom basiert, der sich auf ein erstes chirurgisches Instrument bezieht, wenn der erste Benutzer das erste chirurgische Instrument verwendet;
wobei die zweite interaktive Überlagerung auf einem zweiten Datenstrom basiert, der sich auf ein zweites chirurgisches Instrument bezieht, wenn der zweite Benutzer das zweite chirurgische Instrument verwendet;
wobei die erste interaktive Überlagerung aktualisiert wird, um auf dem zweiten Datenstrom zu basieren, wenn der erste Benutzer das zweite chirurgische Instrument verwendet; und
wobei die Vielzahl von Datenströmen den ersten Datenstrom und den zweiten Datenstrom umfasst.

15. Chirurgisches System nach Anspruch 10, wobei der Risikoschwellenwert auf der Häufigkeit des erkannten Fehlers, der Überschreitung des erwarteten Bereichs durch den erkannten Fehler, einem Kommunikationsverlust mit dem ersten chirurgischen Instrument oder einer Kombination davon basiert.

16. Chirurgisches System nach Anspruch 10, wobei der chirurgische Knotenpunkt so konfiguriert ist, dass er eine Sperrung des ersten chirurgischen Instruments bewirkt, wenn der erkannte Fehler den Risikoschwellenwert überschreitet.

17. System nach Anspruch 10, das ferner ein chirurgisches Gerät umfasst, das so konfiguriert ist, dass es mit dem chirurgischen Knotenpunkt gepaart wird;
wobei durch die automatische Kopplung eines chirurgischen Geräts mit dem chirurgischen Knotenpunkt eine erste Sicherheitsstufe erreicht wird;
wobei durch manuelles Koppeln des chirurgischen Geräts mit dem chirurgischen Knotenpunkt eine zweite Sicherheitsebene erreicht wird;
wobei der chirurgische Knotenpunkt dazu konfiguriert ist, einen zwischen dem chirurgischen Gerät und dem chirurgischen Knotenpunkt übertragenen Datenstrom zu authentifizieren; und
wobei der Datenstrom einer aus der Vielzahl von Datenströmen ist.

18. System nach Anspruch 10, ferner umfassend ein chirurgisches Gerät mit einem Quick-Response-Code (QR-Code), wobei das chirurgische Gerät so konfiguriert ist, dass es zunächst Geräteauthentifizierungsdaten an den chirurgischen Knotenpunkt sendet;
wobei der chirurgische Knotenpunkt so konfiguriert ist, dass er den QR-Code scannt, um zweite Geräteauthentifizierungsdaten zu empfangen;
wobei der chirurgische Knotenpunkt dazu konfiguriert ist, die ersten Geräteauthentifizierungsdaten und die zweiten Geräteauthentifizierungsdaten zu vergleichen;
wobei die erste Augmented-Reality-Anzeige dazu konfiguriert ist, eine Benachrichtigung basierend auf den ersten Geräteauthentifizierungsdaten und den zweiten Geräteauthentifizierungsdaten anzuzeigen; und
wobei die ersten Geräteauthentifizierungsdaten einer der Vielzahl von Datenströmen sind.

## Revendications

1. Procédé permettant d'afficher des calques interactifs pour de multiples utilisateurs d'un système chirurgical, le procédé comprenant :
la réception, par un contrôleur chirurgical central, d'une pluralité de flux de données apparentés à une procédure chirurgicale ;
le couplage en communication d'un premier dispositif d'affichage en réalité augmentée avec un contrôleur chirurgical central ;
la liaison, par le contrôleur chirurgical central, du premier dispositif d'affichage en réalité augmentée à un premier utilisateur ;
l'affichage, par le premier dispositif d'affichage en réalité augmentée, d'un premier calque interactif personnalisé pour le premier utilisateur en fonction d'au moins l'un parmi la pluralité de flux de données ;
la transmission, par un premier instrument chirurgical, d'un flux de données de premier instrument chirurgical au contrôleur chirurgical central, dans lequel le flux de données de premier instrument chirurgical est l'un parmi la pluralité de flux de données apparentés à la procédure chirurgicale ;
la détection, par le contrôleur chirurgical central, d'une erreur apparentée au flux de données de premier instrument chirurgical ; et
le fait d'amener, par le contrôleur chirurgical central, le premier calque de données interactif à comporter une alerte en fonction de l'erreur détectée dépassant un seuil de niveau de risque.

2. Procédé selon la revendication 1, comprenant en outre :
le couplage en communication d'un second dispositif d'affichage en réalité augmentée au contrôleur chirurgical central ;
la liaison, par le contrôleur chirurgical central, du second dispositif d'affichage en réalité augmentée à un second utilisateur ; et
l'affichage, par le second dispositif d'affichage en réalité augmentée, d'un second calque interactif personnalisé pour le second utilisateur en fonction d'au moins l'un parmi la pluralité de flux de données.

3. Procédé selon la revendication 2, comprenant en outre l'affichage simultané du premier calque interactif par le premier dispositif d'affichage en réalité augmentée et du second calque interactif par le second dispositif d'affichage en réalité augmentée ;
dans lequel le premier calque interactif et le second calque interactif sont en fonction du même flux de données de la pluralité de flux de données.

4. Procédé selon la revendication 3, comprenant en outre l'affichage du premier calque interactif et du second calque interactif différemment pour le premier utilisateur et le second utilisateur en fonction des préférences respectives du premier utilisateur et du second utilisateur.

5. Procédé selon la revendication 2, comprenant en outre :
l'affichage, par le premier dispositif d'affichage en réalité augmentée, du premier calque interactif en fonction d'un premier flux de données apparenté à un premier instrument chirurgical lorsque le premier utilisateur est en train d'utiliser le premier instrument chirurgical ;
l'affichage, par le second dispositif d'affichage en réalité augmentée, du second calque interactif en fonction d'un second flux de données apparenté à un second instrument chirurgical lorsque le second utilisateur est en train d'utiliser le second instrument chirurgical ; et
la mise à jour, par le premier dispositif d'affichage en réalité augmentée, du premier calque interactif pour qu'il soit en fonction du second flux de données lorsque le premier utilisateur est en train d'utiliser le second instrument chirurgical ;
dans lequel la pluralité de flux de données comprend le premier flux de données et le second flux de données.

6. Procédé selon la revendication 1, dans lequel le seuil de niveau de risque est en fonction d'une fréquence de l'erreur détectée, de l'erreur détectée dépassant une plage attendue, d'une perte de communication avec le premier instrument chirurgical, ou d'une combinaison de ceux-ci.

7. Procédé selon la revendication 6, comprenant en outre le fait d'amener, par le contrôleur chirurgical central, un verrouillage du premier instrument chirurgical en fonction de l'erreur détectée dépassant le seuil de niveau de risque.

8. Procédé selon la revendication 1, comprenant en outre :
l'appairage automatique, par le contrôleur chirurgical central, d'un dispositif chirurgical au contrôleur chirurgical central pour réaliser un premier niveau de sécurité ou l'appairage manuel, par un utilisateur, du dispositif chirurgical au contrôleur chirurgical central pour réaliser un second niveau de sécurité ; et
l'authentification, par le contrôleur chirurgical central, d'un flux de données transmis entre le dispositif chirurgical et le contrôleur chirurgical central ;
dans lequel le flux de données est l'un parmi la pluralité de flux de données.

9. Procédé selon la revendication 1, comprenant en outre :
le balayage, par le contrôleur chirurgical central, d'un code de réponse rapide (QR) d'un dispositif chirurgical pour recevoir des premières données d'authentification de dispositif ;
la transmission, par le dispositif chirurgical, de secondes données d'authentification de dispositif au contrôleur chirurgical central, dans lequel les secondes données d'authentification de dispositif sont l'un parmi la pluralité de flux de données ;
la comparaison, par le contrôleur chirurgical central, des premières données d'authentification de dispositif aux secondes données d'authentification de dispositif ; et
l'affichage, par le premier affichage en réalité augmentée, d'une notification en fonction de la comparaison des premières données d'authentification de dispositif aux secondes données d'authentification de dispositif.

10. Système chirurgical permettant d'afficher des calques interactifs pour de multiples utilisateurs, le système comprenant :
un contrôleur chirurgical central configuré pour recevoir une pluralité de flux de données apparentés à une procédure chirurgicale ; et
un premier dispositif d'affichage en réalité augmentée couplé en communication au contrôleur chirurgical central, dans lequel le premier dispositif d'affichage en réalité augmentée est lié à un premier utilisateur ;
dans lequel le premier dispositif d'affichage en réalité augmentée affiche un premier calque interactif personnalisé pour le premier utilisateur en fonction d'au moins l'un parmi la pluralité de flux de données,
un premier instrument chirurgical configuré pour transmettre un flux de données de premier instrument chirurgical au contrôleur chirurgical central, dans lequel le flux de données de premier instrument chirurgical est l'un parmi la pluralité de flux de données apparentés à la procédure chirurgicale ;
dans lequel le contrôleur chirurgical central est configuré pour détecter une erreur apparentée au flux de données de premier instrument chirurgical ; et
dans lequel le contrôleur chirurgical central est configuré pour amener le premier calque de données interactif à comporter une notification en fonction de l'erreur détectée dépassant un seuil de niveau de risque.

11. Système chirurgical selon la revendication 10, comprenant en outre un second dispositif d'affichage en réalité augmentée couplé en communication au contrôleur chirurgical central, dans lequel le second dispositif d'affichage en réalité augmentée est lié à un second utilisateur ;
dans lequel le second dispositif d'affichage en réalité augmentée affiche un second calque interactif personnalisé pour le second utilisateur en fonction d'au moins l'un parmi la pluralité de flux de données.

12. Système chirurgical selon la revendication 11, dans lequel le premier calque interactif et le second calque interactif sont en fonction du même flux de données de la pluralité de flux de données ; et
dans lequel le premier dispositif d'affichage en réalité augmentée et le second dispositif d'affichage en réalité augmentée affichent le premier calque interactif et le second calque interactif simultanément.

13. Système chirurgical selon la revendication 12, dans lequel le premier calque interactif et le second calque interactif sont affichés différemment pour le premier utilisateur et le second utilisateur en fonction des préférences respectives du premier utilisateur et du second utilisateur.

14. Système chirurgical selon la revendication 11, dans lequel le premier calque interactif est en fonction d'un premier flux de données apparenté à un premier instrument chirurgical lorsque le premier utilisateur est en train d'utiliser le premier instrument chirurgical ;
dans lequel le second calque interactif est en fonction d'un second flux de données apparenté à un second instrument chirurgical lorsque le second utilisateur est en train d'utiliser le second instrument chirurgical ;
dans lequel le premier calque interactif se met à jour pour être en fonction du second flux de données lorsque le premier utilisateur est en train d'utiliser le second instrument chirurgical ; et
dans lequel la pluralité de flux de données comprend le premier flux de données et le second flux de données.

15. Système chirurgical selon la revendication 10, dans lequel le seuil de niveau de risque est en fonction d'une fréquence de l'erreur détectée, de l'erreur détectée dépassant une plage attendue, d'une perte de communication avec le premier instrument chirurgical, ou d'une combinaison de ceux-ci.

16. Système chirurgical selon la revendication 10, dans lequel le contrôleur chirurgical central est configuré pour amener un verrouillage du premier instrument chirurgical en fonction de l'erreur détectée dépassant le seuil de niveau de risque.

17. Système selon la revendication 10, comprenant en outre un dispositif chirurgical configuré pour être apparié au contrôleur chirurgical central ;
dans lequel un appairage automatique du dispositif chirurgical au contrôleur chirurgical central réalise un premier niveau de sécurité ;
dans lequel un appairage manuel du dispositif chirurgical au contrôleur chirurgical central réalise un second niveau de sécurité ;
dans lequel le contrôleur chirurgical central est configuré pour authentifier un flux de données transmis entre le dispositif chirurgical et le contrôleur chirurgical central ; et
dans lequel le flux de données est l'un parmi la pluralité de flux de données.

18. Système revendication 10, comprenant en outre un dispositif chirurgical comprenant un code de réponse rapide (QR), le dispositif chirurgical étant configuré des premières données d'authentification de dispositif au contrôleur chirurgical central ;
dans lequel le contrôleur chirurgical central est configuré pour balayer le code QR pour recevoir des secondes données d'authentification de dispositif ;
dans lequel le contrôleur chirurgical central est configuré pour comparer les premières données d'authentification de dispositif et les secondes données d'authentification de dispositif ;
dans lequel le premier affichage en réalité augmentée est configuré pour afficher une notification en fonction des premières données d'authentification de dispositif et des secondes données d'authentification de dispositif ; et
dans lequel les premières données d'authentification de dispositif sont l'un parmi la pluralité de flux de données.
